# EUROPEAN PATENT APPLICATION

(11) **EP 2 492 354 A1**
(43) Date of publication of application: **29.08.2012**
(21) Application number: 11001498.2
(22) Date of filing: 23.02.2011
(51) Int. Cl.: C12Q 1/68

(54) **Method for identifying compounds useful in the treatment of photosensitive reflex epilepsy**

(71) Applicant: Institut National De La Recherche Agronomique (INRA), 75338 Paris Cedex 07 (FR)
(72) Inventor: Pitel, Frédérique, 31460 Beauville (FR)
(74) Representative: Barbot, Willy

(57) **Abstract**

The present invention relates to a method for diagnosing photosensitive reflex epilepsy in a subject, which comprises the steps of analyzing a biological sample from said subject by i) detecting the presence of a mutation in the Synaptic Vesicle protein 2 A (SV2A) gene leading to an underexpression or absence of expression of said gene, and/or ii) determining the expression level of the SV2A gene, wherein said mutation and/or expression level of the SV2A gene is indicative of photosensitive reflex epilepsy in said subject. The present invention also relates to a method for identifying a compound useful in the treatment of photosensitive reflex epilepsy, the method comprising the steps of a) administering said compound to a non-human photosensitive epileptic animal, preferably a chicken, presenting a mutation of the Synaptic Vesicle protein 2 A (SV2A) gene leading to the underexpression of said gene, b) measuring the responsiveness of said non-human treated animal to Intermittent Light Stimulation (ILS), c) comparing the responsiveness to intermittent light stimulation of said treated animal with the responsiveness of a non-human non treated animal to ILS, d) selecting a compound for which said treated animal presents an absent or diminished photosensitive reflex epileptic phenotype compared to a non treated animal.

## Description

### FIELD OF THE INVENTION

The present invention relates to methods for the diagnosis of photosensitive reflex epilepsy, methods for identifying compounds useful in the treatment of photosensitive reflex epilepsy and use of LEVETIRACETAM for the treatment of said photosensitive reflex epilepsy.

### BACKGROUND OF THE INVENTION

Epilepsy is a common brain disorder characterized by recurrent seizures. A seizure can be defined as an abnormal, excessive and synchronous neuronal activity in the brain, resulting in a temporary disturbance of motor, sensory, or mental function.

Epilepsy has many possible causes, including illness, brain injury and abnormal brain development, but in many cases, the cause is unknown.

There are several types of epilepsy syndromes, each being characterized by its own combination of seizure type, typical age of onset, abnormal electroencephalogram, treatment, and prognosis. They have been sorted in different classifications regarding the types of seizures, the causes of epilepsy or the symptoms observed. The classification regarding the type of seizures is based on the repartition in three classes comprising: localization-related epilepsies wherein seizures are produced in a small portion of the brain, generalized epilepsies wherein seizures occur in the whole brain and finally epilepsies of unknown localization which remain unclear whether the seizures arise from a portion of the brain or from more widespread circuits. In addition to the localized and generalized groups, there are further subdivisions into idiopathic (associated to a genetically transmitted trait), symptomatic (identifiable cause), or cryptogenic (hidden cause) epilepsies. The classification system of epilepsies appears to be complex and many syndromes remains not adequately defined.

Reflex Epilepsy is a group of epilepsy syndromes characterized by seizures which are generated by a person's acuteness to sensor stimulation caused by the environment such as certain lights, sounds, music, movements or some more complex events such as reading, writing, doing arithmetic, or even thinking about specific topics.

Within said reflex epilepsy, the photosensitive epilepsy appears to be the most common and most of all more frequent reflex epilepsy regarding the increasing use of television, video games and computers in common lives. It occurs in 1 per 4000 individuals, with a higher incidence in children between 7 and 19 years of age (10% of epileptic children) than in adults (5% of epileptic adults).

Photosensitive reflex epilepsy is caused by the combination of individual genetic sensitivity of predisposed subjects with an external stimulus, generally an intermittent light stimulus such as stroboscopic light. Photosensitive epilepsy is characterized by an abnormal electroencephalographic reaction showing spike-and-wave discharges and seizures triggered by Intermittent Light Stimulation (ILS), which are ranging from a simple photoparoxysmal electrical discharge (PPR) to generalized seizures. In human, the most effective frequency for the start of a photosensitive reflex epilepsy seizure is of 15 flashes of lightning per second, which can be also associated to some particular moieties or colors.

Diagnosis of photosensitive reflex epilepsy is currently made by analyzing the correlation between exposure to specific visual stimuli and seizure activity. More precise investigation can be carried out by combining an electroencephalogram with a device producing Intermittent Light Stimulation (ILS), but the absence of identified genes responsible for photosensitive reflex epilepsy does not allow the proceeding of a simple genetic diagnosis.

Though several studies brought a strong support for a genetic etiology for photosensitive epilepsy, very few information on the genetic factors responsible for such a predisposition are available. The difficulties of phenotype characterization, its variable expression in particular because of the age, the obviousness of the existence of several different genes in certain studies and the few availability of informative families contribute to the difficulty of identifying in human the genes which are responsible for the photosensitive epilepsy and which remain unknown.

Therefore, the use of animal models for studying photosensitive reflex epilepsy is essential for the search of new effective antiepileptic drugs. Photosensitive reflex epilepsy has been studied in a primate, *Papio papio,* and in the chicken. Several studies brought a strong support for a genetic etiology, but no causative gene or mutation has yet been identified to be responsible for photosensitive reflex epilepsy.

### SUMMARY OF THE INVENTION

For the first time, the inventors have surprisingly demonstrated in an animal that photosensitive reflex epilepsy has a genetic base which is associated with the gene encoding the Synaptic Vesicle protein 2A, a protein associated with neurological disorders. Indeed they have identified that the significant decrease of the SV2A gene expression in photosensitive reflex epileptic animals appeared to be due to a mutation in the SV2A gene provoking abnormal splicing events. A similar mutation has been identified in the intron 6 of the human SV2A protein by the University of Bonn, the presence of said mutation being responsible for modifying the response of a patient to an antiepileptic treatment, as it is described in the international application WO2007036552A1. However, said mutation has not been demonstrated for being responsible for the Photosensitive Reflex Epilepsy.

Therefore, the discovery of the inventors enables to consider for the first time methods of diagnosis of photosensitive reflex epilepsy in subjects thought to present such specific epilepsy by analysing the SV2A gene expression.

The determination of said genetic base also enables the identification of compounds useful in the treatment of photosensitive reflex epilepsy. Said identification can be realized by using an animal model presenting a mutation in the SV2A gene leading to an underexpression of said gene.

Especially, the inventors have tested the anticonvulsant effect of the LEVETIRACETAM in the animal model according to the invention presenting an under-expression of SV2A gene, whereas the LEVETIRACETAM was known for the treatment of neurological disorders by its binding to the same SV2A protein (WO2004/051222 They demonstrated that the treated animals present significantly reduced symptoms of said photosensitive reflex epilepsy, surprisingly. Thus, the LEVETIRACETAM may act on this photosensitive reflex epilepsy even with a drastically reduced level of SV2A protein, or by another unknown target being different from the previously identified SV2A protein. Therefore, the invention also relates to the use of LEVETIRACETAM for the treatment of photosensitive epilepsy.

### DETAILED DESCRIPTION OF THE INVENTION

In a first aspect, the invention provides a method for diagnosing photosensitive reflex epilepsy in a subject, which comprises the steps of analyzing a biological sample from said subject by:
i) Detecting the presence of a mutation in the Synaptic Vesicle protein 2 A (SV2A) gene leading to an underexpression or absence of expression of said gene, and/or
ii) Determining the expression level of the SV2A gene,

Wherein said mutation and/or said expression level of said gene is indicative of photosensitive reflex epilepsy in said subject.

As defined previously, photosensitive reflex epilepsy is a form of reflex epilepsy that is triggered by visual stimuli, such as flickering or high contrast oscillating patterns. It is characterized by an abnormal electroencephalographic reaction showing spike-and-wave discharges and seizures triggered by Intermittent Light Stimulation (ILS).

As used herein, the term "subject" refers to an animal, preferably a human.

Said subject may be healthy, but the method of the invention is particularly useful for testing a subject thought to develop or to be predisposed to developing photosensitive reflex epilepsy. In that case, the method of the invention enables to confirm that said subject develops or is predisposed for developing photosensitive reflex epilepsy.

Said subject may be also diagnosed as suffering from photosensitive reflex epilepsy and being treated by a specific treatment, wherein the step of ii) determining the expression level of the SV2A gene is indicative of the efficiency of said specific treatment on said subject.

The Synaptic Vesicular protein 2 A (SV2A) is an integral membrane protein isoform belonging to the family of SV2 proteins. The SV2A protein is expressed ubiquitously throughout the brain as well in secretory granules of endocrine cells and is the only isoform present in the GABAergic neurons.

SV2 proteins are one of the most common proteins of synaptic vesicles, and have been implicated in the control of calcium-mediated exocytosis of synaptic vesicles. SV2 proteins have also been shown to be expressed in endocrine cells and, along with the additional synaptic vesicle membrane integral proteins p38 and p65, have been demonstrated to be present in endocrine dense core granule membranes.

SV2 proteins have relatively short free N-and C-termini and short loops connecting the Tm segments. Two notable exceptions, however, are the long cytoplasmic loop between transmembrane regions 6 and 7 and the intravesicular loop between transmembrane regions 7 and 8 (which contains 3 N-glycosylation sites).

The human SV2A gene is referenced with the accession number NP_055664, and its cDNA (Accession number NM_014849, SEQ ID n°1) is coding for a protein of 742 amino acids (Accession number NP_055664, SEQ ID n°2).

As used herein, the expression "biological sample" refers to buccal swab, fluids, neuronal tissue, endocrinal tissue and excretions such as for example, sputum, induced sputum, blood, serum, plasma, urine.

In a preferred embodiment, the method comprises the step of detecting the presence of a mutation in the SV2A gene leading to an underexpression or absence of expression of said gene.

In a still more preferred embodiment, the method comprises the step of detecting the presence of a mutation in the SV2A gene leading to an absence of expression of said gene.

As used herein, the term "mutations" corresponds to any modification in the sequence of the original nucleic acid sequence. These mutations comprise small-scale mutations, or large scale mutations. Small scale mutations are those affecting a gene in one or a few nucleotides, including point mutations, insertions or deletions of one or more extra nucleotides in the DNA. Point mutations can be silent, missense and nonsense mutation. Large scale mutation in the genomic structure, such as gene duplications, deletions, or mutations whose effect is to juxtapose previously separate pieces of DNA, potentially bringing together separate genes to form functionally distinct fusion genes. These last mutations include chromosomal translocations, interstitial deletions, chromosomal inversions and loss of heterezygosity.

Preferably, only a biological sample containing cells including genomic DNA (or optionally RNA) from the subject to be tested is required.

In a particular embodiment, the in vitro method of the invention aims to detect mutation included in the group consisting of deletion, and point mutations corresponding to missense mutations and nonsense mutations.

The inventors have established that the existence of such mutations is associated with photosensitive reflex epilepsy.

For deletion, said deletion preferably results in the absence of expression of the SV2A protein or in the expression of a truncated SV2A protein.

For missense mutation, said missense mutation is preferably located in the open reading frame of the SV2A protein.

Examples of missense mutations comprise:
- missense mutation located in the region of SEQ ID N°3 at the level of the nucleic acid 2418 of the human SV2A mRNA transcript, corresponding to the amino acid 643 of the protein (Accession number rs1801870). When X corresponds to a Thymine (T), the SV2A gene is not under-expressed whereas when X corresponds to an Adenine (A), the SV2A gene is under-expressed.
   SEQ ID N°3 corresponds to an exonic region of the SV2A gene.
- missense mutation located in the region of SEQ ID N°4 at the level of the nucleic acid 2056 of the human SV2A mRNA transcript , corresponding to the amino acid 522 of the protein (Accession number rs 111800602). When X corresponds to an Adenine (A), the SV2A gene is not under-expressed whereas when X corresponds to a Guanine (G) the SV2A gene is under-expressed.
   SEQ ID N°4 corresponds to an exonic region of the SV2A gene.
- missense mutation located in the region of SEQ ID N°5 at the level of the nucleic acid 1821 of the human SV2A mRNA transcript, corresponding to the amino acid 444 of the protein (Accession number rs117711516). When X corresponds to a Cytosine (C), the SV2A gene is not under-expressed whereas when X corresponds to a Thymine (T) the SV2A gene is under-expressed.
   SEQ ID N°5 corresponds to an exonic region of the SV2A gene.
- missense mutation located in the region of SEQ ID N°6 at the level of the nucleic acid 1162 of the human SV2A mRNA transcript, corresponding to the amino acid 643 of the protein (Accession number rsl 117711516). When X corresponds to a Guanine (G), the SV2A gene is not under-expressed whereas when X corresponds to a Thymine (T) the SV2A gene is under-expressed.
   SEQ ID N°6 corresponds to an exonic region of the SV2A gene.

The person skilled in the art can easily identify other mutations on the SV2A gene of other animals such as in mice, wherein the SV2A gene knock down or knock out leads to the underexpression or absence of expression of said gene.

Typical techniques for detecting the presence of a mutation may include restriction fragment length polymorphism, hybridization techniques, DNA sequencing, exonuclease resistance, microsequencing, solid phase extension using ddNTPs, extension in solution using ddNTPs, oligonucleotide ligation assays, methods for detecting single nucleotide polymorphisms such as dynamic allele-specific hybridization, ligation chain reaction, mini-sequencing, DNA "chips", allele-specific oligonucleotide hybridization with single or dual-labelled probes merged with PCR or with molecular beacons, and others.

Advantageously, the mutation is detected on the DNA or cDNA of the SV2A gene by PCR and either sequencing or genotyping methods, all of them being well known for the skilled person.

In molecular biology and bioinformatics, a SNP array is a type of DNA microarray which is used to detect polymorphisms within a population. The basic principles of SNP array are the same as the DNA microarray. These are the convergence of DNA hybridization, fluorescence microscopy, and solid surface DNA capture. The three mandatory components of the SNP arrays are: i) the array that contains immobilized nucleic acid sequences or target; ii) one or more labeled Allele specific oligonucleotide (ASO) probes; and iii) a detection system that records and interprets the hybridization signal (see in Sheils, O., Finn, S. and O'Leary J. (2003) "Nucleic acid microarray: an overview." Current Diagnostic Pathology. 9:155-158).

In another preferred embodiment of the invention, the method comprises the step of analyzing the expression of the Synaptic Vesicle protein 2 A (SV2A) gene.

According to the results obtained by the inventors, the under-expression or the absence of expression of the SV2A gene is associated with photosensitive reflex epilepsy.

Methods for analyzing the expression of a gene are well known for the man skilled in the art.

In a particular embodiment of the invention, the expression of the SV2A gene is assessed by analyzing the expression of mRNA transcript or mRNA precursors, such as nascent RNA, of said gene.

Preferably, only a biological sample containing cells including DNA and/or RNA from the subject to be tested is required.

Such analysis can be assessed by preparing mRNA/cDNA from cells in a biological sample from a subject, and hybridizing the mRNA/cDNA with a reference polynucleotide. The prepared mRNA/cDNA can be used in hybridization or amplification assays that include, but are not limited to, Southern or Northern analyses, polymerase chain reaction analyses, such as quantitative PCR (TAQMAN), and probes arrays such as GENECHIP™ DNA Arrays (AFFYMETRIX).

Advantageously, the analysis of the expression level of mRNA transcribed from the SV2A gene involves the process of nucleic acid amplification, e. g., by RT-PCR (the experimental embodiment set forth in U. S. Patent No. 4,683, 202), ligase chain reaction (BARANY, Proc. Natl. Acad. Sci. USA, vol.88, p: 189-193, 1991), self sustained sequence replication (GUATELLI et al., Proc. Natl. Acad. Sci. USA, vol.87, p: 1874-1878, 1990), transcriptional amplification system *(*KWOH et al., 1989, Proc. Natl. Acad. Sci. USA, vol.86, p: 1173-1177, 1989), Q-Beta Replicase *(*LIZARDI et al., Biol. Technology, vol.6, p: 1197, 1988), rolling circle replication (U. S. Patent No. 5,854, 033) or any other nucleic acid amplification method, followed by the detection of the amplified molecules using techniques well known to those of skill in the art. These detection schemes are especially useful for the detection of nucleic acid molecules if such molecules are present in very low numbers. As used herein, amplification primers are defined as being a pair of nucleic acid molecules that can anneal to 5' or 3'regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers.

In another particular embodiment, the expression of the SV2A gene is assessed by analyzing the expression of the SV2A protein translated from said gene.

Preferably, only a biological sample containing neuronal or endocrinal cells from the subject to be tested is required.

Such analysis can be assessed using an antibody (e.g., a radio-labeled, chromophorelabeled, fluorophore-labeled, or enzyme-labeled antibody), an antibody derivative (e.g., an antibody conjugate with a substrate or with the protein or ligand of a protein of a protein/ligand pair (e.g., biotin-streptavidin)), or an antibody fragment (e.g., a single-chain antibody, an isolated antibody hypervariable domain, *etc.)* which binds specifically to the protein translated from the SV2A gene. Said analysis can be assessed by a variety of techniques well known by one of skill in the art including, but not limited to, enzyme immunoassay (EIA), radioimmunoassay (RIA), Western blot analysis and enzyme linked immunoabsorbant assay (ELISA).

A skilled person in the art is able to find antibodies directed against SV2A protein.

Polyclonal antibodies can be prepared by immunizing a suitable animal, such as mouse, rabbit or goat, with the SV2A protein or a fragment thereof (e.g., at least 10 or 15 amino acids). The antibody titer in the immunized animal can be monitored over time by standard techniques, such as with an ELISA using immobilized polypeptide. At an appropriate time after immunization, e.g., when the specific antibody titers are highest, antibody producing cells can be obtained from the animal and used to prepare monoclonal antibodies (mAb) by standard techniques, such as the hybridoma technique originally described by KOHLER and MILSTEIN (Nature, vol.256, p:495-497, 1975), the human B cell hybridoma technique (KOZBOR et al., Immunol., vol.4, p: 72, 1983), the EBV- hybridoma technique (COLE et al., In Monoclonal Antibodies and Cancer Therapy, Alan R. Liss,Inc., p: 77-96, 1985) or trioma techniques. The technology for producing hybridomas is well known (see generally Current Protocols in Immunology, COLIGAN et al. ed., John Wiley & Sons, New York, 1994). Hybridoma cells producing the desired monoclonal antibody are detected by screening the hybridoma culture supernatants for antibodies that bind the polypeptide of interest, e.g., using a standard ELISA.

Commercial SV2A antibodies are available, as an example, one can cite the ones available from ABCAM, ABNOVA CORPORATION, ATLAS ANTIBODIES, ACRIS ANTIBODIES GMBH, AVIVA SYSTEMS BIOLOGY, EVEREST BIOTECH, GENWAY BIOTECH, INC., NOVUS BIOLOGICALS, RAYBIOTECH INC., SIGMA-ALDRICH or SYNAPTIC SYSTEMS GMBH.

As previously mentioned, mutations in the SV2A gene may trigger the under-expression or absence of expression of the SV2A protein.

As used herein, the "under-expression" of a polypeptide occurs when the transcription and/or the translation of the gene is affected by the mutation, leading to an expression level in a biological sample that is lower than the standard error of the assay employed to assess expression, and is preferably at least 20% inferior to the normal level of expression of said gene, preferably at least 50% inferior to the normal level of expression of said gene, and most preferably at least 80% inferior to the normal level of expression of said gene.

As used herein, the "absence of expression" of a polypeptide occurs when the transcription of the gene is prevented due to the mutation, leading to a lack of expression of the protein and therefore to an expression level in a biological sample that is null compared to the normal level of expression of said gene.

Therefore, the method of the invention may comprise the step of comparing the level of expression of the SV2A gene in a biological sample from a subject with the expression level of said gene in a control (i.e., normal expression level). A significantly lower level of expression or an absence of expression of said gene in the biological sample of a subject as compared to the normal expression level is an indication that the patient has photosensitive reflex epilepsy.

As used herein, a "control" corresponds preferably to a control sample from a subject who does not have photosensitive epilepsy.

Thus, the "normal" level of expression of the SV2A gene is the level of expression of said gene in a biological sample of a non photosensitive reflex epileptic subject.

Analyzing the normal expression of the SV2A gene may be assessed by any of a wide variety of well-known methods for detecting expression of a transcribed nucleic acid or translated protein as previously described.

In a second aspect, the present invention refers to a kit for diagnosing photosensitive reflex epilepsy in a subject comprising at least one nucleic acid probe or oligonucleotide or at least one antibody, which can be used in a method as defined in the present invention, for detecting the presence of a mutation in the SV2A gene and/or analyzing the expression of the SV2A gene.

Preferably, the oligonucleotide is at least one PCR primer pair, preferably a set of PCR primers is provided, which allows to sequence the corresponding SV2A gene or fragment thereof. The skilled person readily provides such an oligonucleotide or set of PCR primers which allows to amplify a region of the SV2A gene, provided that the nucleic acid sequence of SV2A is well known (Accession number NM_014849, SEQ ID N°1) (Current Protocols in Molecular Biology; edited by Fred M. Ausubel et al., supra).

As used herein, the term "kit" refers to any delivery system for delivering materials. In the context of reaction assays, such delivery systems include systems that allow for the storage, transport, or delivery of reaction reagents (e.g., oligonucleotides, enzymes, etc. in the appropriate containers) and/or supporting materials (e.g., buffers, written instructions for performing the assay etc.) from one location to another. For example, kits include one or more enclosures (e.g., boxes) containing the relevant reaction reagents and/or supporting materials. As used herein, the term "fragmented kit" refers to delivery systems comprising two or more separate containers that each contains a subportion of the total kit components. The containers may be delivered to the intended recipient together or separately. For example, a first container may contain an enzyme for use in an assay, while a second container contains oligonucleotides. The term "fragmented kit" is intended to encompass kits containing Analyte specific reagents (ASR's) regulated under section 520(e) of the Federal Food, Drug, and Cosmetic Act, but are not limited thereto. Indeed, any delivery system comprising two or more separate containers that each contains a subportion of the total kit components are included in the term "fragmented kit." In contrast, a "combined kit" refers to a delivery system containing all of the components of a reaction assay in a single container (e.g., in a single box housing each of the desired components). The term "kit" includes both fragmented and combined kits.

The present kits can also include one or more reagents, buffers, hybridization media, nucleic acids, primers, nucleotides, probes, molecular weight markers, enzymes, solid supports, databases, computer programs for calculating dispensation orders and/or disposable lab equipment, such as multi-well plates, in order to readily facilitate implementation of the present methods. Enzymes that can be included in the present kits include nucleotide polymerases and the like. Solid supports can include beads and the like whereas molecular weight markers can include conjugatable markers, for example biotin and streptavidin or the like.

In one embodiment, the kit is made up of instructions for carrying out the method described herein for diagnosing photosensitive epilepsy in a subject. The instructions can be provided in any intelligible form through a tangible medium, such as printed on paper, computer readable media, or the like.

Still a further aspect of the present invention refers to the use, for diagnosing photosensitive epilepsy, of the abovementioned kit comprising at least one nucleic acid probe or oligonucleotide or at least one antibody, which can be used in a method as defined for detecting the presence of a mutation in the SV2A gene and/or analysing the expression of the SV2A gene.

A third aspect of the invention relates to a method for identifying a compound useful in the treatment of photosensitive reflex epilepsy, the method comprising the steps of
(a) Administering said compound to a non-human photosensitive reflex epileptic animal presenting a mutation in the Synaptic Vesicle protein 2 A (SV2A) gene leading to an underexpression of said gene;
(b) Measuring the responsiveness of said non-human treated animal to Intermittent Light Stimulation (ILS);
(c) Comparing the responsiveness to intermittent light stimulation of said treated animal with the responsiveness of a non-human non treated animal to ILS;
(d) Selecting a compound for which said treated animal presents an absent or reduced photosensitive reflex epileptic phenotype compared to a non treated animal.

By "compound" or "test compound", one should understand compounds of different nature, structure and origin, particularly biological compounds, nuclear factors, cofactors, and the like, chemical, synthetic compounds and the like, which are tested for their capacity of diminishing or suppressing the symptoms of photosensitive epilepsy. Advantageously, said compound is selected in the group comprising peptides, polypeptides, small-molecules, nucleic acids, lipids, and carbohydrates.

The concentration of said test compound can be adjusted by the skilled person according to the characteristics of said compound (its toxicity, ability to penetrate cells, etc.), the length of the incubation period, etc. Generally, the animals are exposed to concentrations of test compounds ranging from 1 mg/ml to 1000 mg/ml. Of course it is possible to test other concentrations without deviating from the invention, and also to test simultaneously different test compound concentrations.

The animal used in the method for identifying a compound useful in the treatment of photosensitive reflex epilepsy is a non-human animal, preferably a chicken.

Said non-human animal of the invention has been previously diagnosed with photosensitive reflex epilepsy.

Preferably, said non-human animal has been identified with Intermittent Light Stimulation seizures at birth.

The term "Intermittent Light Stimulation" (ILS) refers to a form of visual stimulation applied on a subject for investigating anomalous brain activity generally in combination with electroencephalography. Stimuli which can be used are selected in the group comprising flashing lights that are high in luminance contrast, such as bright flashes of light alternating with darkness or white bars against a black background, and flashing patterns such as rapidly changing or alternating images or colors.

Preferably, said non-human animal has been phenotypically identified with Intermittent Light Stimulation seizures at birth under epileptogenic frequency of 14 flashes of lightning per second, which is the best effective frequency for the start of an epilepsy crisis for the chicken.

Behaviorally, the types of paroxysmal manifestations are organized in stereotyped phases: preconvulsive and convulsive. The preconvulsive phase starts with an alert which was called "ictal arousal", followed by an ILS-induced neck myoclonus rythmically appearing at the light frequency. The convulsive phase is characterized by self-sustained (persisting after the cessation of the stimulus) generalized convulsions.

The resting electroencephalogram (EEG) of the Fepi chicken (called interictal EEG) consists of continuous paroxysmal discharges of large amplitude spikes and spikes and waves, thus revealing the epileptic predisposition of the homozygous. During seizures, paroxysmal discharges disappear and are replaced by desynchronization of the rhythms.

Unit activity recorded in the prosencephalon shows interictal paroxysmal bursting discharges synchronous to the EEG spikes or spikes and waves. During seizures, bursting is suppressed as EEG desynchronization occurs. In selected mesencephalic structures on the contrary, interictal unit activity is normal, whereas during ILS-induced EEG desynchronization, the neurons show paroxysmal bursting at the light frequency, consistent with the ILS-induced myoclonus.

Furthermore, the non-human animal used for the identification of a compound useful in the treatment of photosensitive reflex epilepsy also has a mutation in the SV2A gene leading to the underexpression of said gene.

As used herein, the "under-expression" of a gene occurs when the transcription of the gene is affected by the mutation, leading to an expression level that is preferably at least 20% inferior to the normal level of expression of said gene, preferably at least 50% inferior to the normal level of expression of said gene, and most preferably at least 80% inferior to the normal level of expression of said gene.

Preferably, the mutation leading to the underexpression of the SV2A gene is located in the acceptor site of the second intron of said gene.

Most preferably, the mutation leading to the underexpression of the SV2A gene in the non-human animal can be identified with the PCR primers of SEQ ID N°7 (5'-TGTCCCTGCTACCCGGG-3') and SEQ ID N°8 (5'-CAGAACAGGAAGGTGCCGTAG-3') according to methods as described previously.

Still most preferably, the mutation leading to the underexpression of the SV2A gene of the chicken (SEQ ID N°9) corresponds to a mutation of a cytosine-cytosine (CC) nucleobase pair into another couple of tyrosine-guanine (TG) nucleobase pair in the acceptor site of the second intron of said SV2A gene at the level of the position 7,188-7,189 of SEQID N°9, said position being also called c581-1CC>TG.

The administration of the compound according to the method for identifying a compound useful in the treatment of photosensitive reflex epilepsy can be operated by any method known in the art, for example orally by feeding or via an intravenous, intramuscular, intraperitoneal, intrasternal, subcutaneous or intraarticular injection or via skin application, etc.

Non-human animals used for the identification of said compound are distributed in different groups receiving different dosages of compound or an absence of compound for a placebo control. Each treatment category preferably includes equal proportions of males and females.

Preferably, non-human animals as defined previously receive the administration of a compound during two weeks.

In another embodiment, the administration of the compound can be preceded by a former step of controlling the non-human animals to Intermittent Light Stimulation. Preferably, said control is operated with ILS at 14 flashes of lightning per second.

The term "responsiveness" in reference to an animal treated with a compound refers to the determination of the animal's response to said compound. Preferably, the responsiveness can be determined by analyzing the phenotype of said animal under Intermittent Light Stimulation.

As used herein the term "phenotype" refers to any observable or biochemical properties of an organism, such as the morphology, the behavior and biochemical or physiological properties of the subject that are produced by the interaction of the genotype with the environment.

The phenotype of photosensitive reflex epileptic animals exposed to Intermittent Light Stimulation comprises an abnormal electroencephalographic reaction and seizures induced by visual stimuli.

The term "seizure" refers to abnormal electrical discharges in the brain resulting from an excessive, abnormal or synchronous neuronal activity therefore leading to an intermittent disturbance in the brain's normal function. Seizures can be ranging from a simple paroxysmal electrical discharge to generalized seizures and can be physically accompanied with myoclonus, i.e. a brief and involuntary twitching of a muscle, or generalized convulsions of the body. The nature and severity of seizures vary from one subject to another, as does the nature of the stimuli that triggers such seizures in said subject.

Therefore, said responsiveness can be measured by simple methods which are well known in the art, such as by determining the occurrence and degree of seizures observed in the treated animal and by analyzing the electroencephalogram of said animal combined to its general comportment.

After the time period of treatment with the compound to be identified, the treatment of the non-human animals is stopped, preferably during two weeks.

The method of the invention further comprises a step of comparing the responsiveness of a non-human treated animal with a non-human non-treated animal to ILS. Said comparison can be realized by comparing the phenotypes of both treated and non treated animals.

The term "treated animal" refers to a non-human animal as defined previously which has been administered the compound to be identified according to the invention.

The term "non-treated animal" refers to an animal of control, i.e. a non-human photosensitive reflex epileptic animal from the same specie than the treated animal but which has not been administered any compound to be identified for a placebo control.

Finally, the method according to the invention comprises a step of selecting a compound for which said treated animal presents an absent or reduced photosensitive reflex epileptic phenotype compared to a non treated animal.

Advantageously, the phenotype of non-human treated animals responding to the treatment with a compound corresponds to a normal electroencephalogram or an electroencephalogram showing fewer variations of discharges accompanied with an absence or diminution of seizures' occurrence under ILS.

In a preferred embodiment, the method of the invention comprises a step of determining the SV2A gene expression before and/or after the administration of said compound.

Preferably, the method of the invention comprises the former step of selecting a non-human photosensitive reflex epileptic animal having a mutation in the SV2A gene leading to the underexpression of said gene. Said selection can be realized by using typical techniques for detecting the presence of a mutation as described previously.

In a still preferred embodiment, the method of the invention comprises a former step of determining the expression level of the SV2A gene in the non-human animals used according to the invention. The expression of the SV2A gene can be assessed by analyzing the expression of mRNA transcript or mRNA precursors, such as nascent RNA, or by analyzing the expression of the SV2A protein translated from said gene with methods as defined previously.

Thus, in another preferred embodiment, the method according to the invention permits to select compounds which can be useful for treating and/or preventing photosensitive epilepsy.

In a fourth aspect, the present invention relates to the use of a non-human animal as defined previously for identifying compounds useful for the treatment of photosensitive reflex epilepsy.

In its broadest meaning, the term "treating" or "treatment" refers to reversing, alleviating, inhibiting the progress of, or preventing the disorder or condition to which such term applies, or one or more symptoms of such disorder or condition.

Another aspect of the invention relates to a compound for the treatment of photosensitive reflex epilepsy, preferably, the LEVETIRACETAM, an analog or a derivative thereof.

The inventors tested the antiepileptic effect of the LEVETIRACETAM compound in the photosensitive reflex epileptic animal model according to the invention. They demonstrated that animals tested with LEVETIRACETAM showed reduced seizure number, delayed myoclonus appearance and reduced seizure duration in response to ILS, the effect partially persisting few days after the injection period.

The LEVETIRACETAM refers to the International Non- proprietary name of the compound (S)-a-ethyl-2-oxo-1-pyrrolidine acetamide as disclosed in the European patent No. 0 162 036 B1. LEVETIRACETAM is a levorotary compound belonging to the pyrrolidine compounds family and is a protective agent for the treatment and prevention of hypoxic and ischemic type aggressions of the central nervous system. LEVETIRACETAM is already known for its anticonvulsant properties in the treatment of Parkinson's disease, epilepsy, dyskenia and other neurological disorders. Although the LEVETIRACETAM has been demonstrated to bind to the SV2A protein at the level of the LEVETIRACETAM Binding Site (LBS) as its therapeutic target, its mechanism of action remains unknown.

The inventors have demonstrated that photosensitive reflex epilepsy in the animal model of the invention is due to a mutation in the SV2A gene leading to a highly reduced expression level of said protein. As the LEVETIRACETAM acts as an anticonvulsant through its binding to the SV2A protein, a highly reduced level of expression of said protein would therefore lead to a predicted diminution of the anticonvulsant properties of LEVETIRACETAM. On the contrary, the inventors established that the administration of LEVETIRACETAM enabled to treat photosensitive epileptic animals, which therapeutic action thus acts through an unidentified therapeutic target in this specific pathology or through an interaction with very small amounts of the SV2A protein.

Therefore, another object of the present invention is the use of a composition comprising LEVETIRACETAM, an analog or a derivative thereof for the treatment of a patient suffering from photosensitive reflex epilepsy.

As used herein, the term "LEVETIRACETAM, analogs or derivatives thereof" includes optionally substituted N-alkylated 2-oxo-pyrrolidine derivatives. Preferably, those compounds are alkyl amides derivatives substituted on the positions 4 and/or 5 of the pyrrolidone ring. Examples of optionally substituted N-alkylated 2-oxo-pyrrolidine derivatives include, but are not limited to, compounds such as those disclosed in international patent application PCT/EP01/01992 such as (2S)-2- [(4S)-4- (2, 2-difluorovinyl) -2-oxopyrrolidinyl] butanamide, (2S)-2- [(4R)-2-oxo-4-propylpyrrolidinyl] butanamide, (2S)-2- [(4S)-2-oxo-4-propylpyrrolidinyl] butanamide, and (2S)-2- [4- (3-azidophenyl)-2-oxopyrrolidin-1- yl] butanamide.

As used herein, the term "LEVETIRACETAM analogs or derivatives thereof" further include optionally substituted N-alkylated 2-oxo-piperidinyl derivatives. Preferably, those compounds are alkyl amides derivatives substituted on the position 4 and/or 5 and/or 6 of the 2-oxo-piperidinyl ring. Examples of optionally substituted N-alkylated 2-oxo-pyrrolidine derivatives include, but are not limited to, compounds such as those disclosed in international patent application PCT/EP02/05503 such as (2S)-2- [5- (iodomethyl)-2-oxo-1- piperidinyl] butanamide, (2S)-2- [5- (azidomethyl)-2-oxo-1-piperidinyl] butanamide, 2- (2-oxo- 5-phenyl-1-piperidinyl] butanamide, (2S)-2- [4- (iodomethyl)-2-oxo-1-piperidinyl] butanamide, and (2S)-2- [4- (2-fluoro-2-methylpropyl)-2-oxo-1-pyrrolidinyl] butanamide.

As used herein, the term "LEVETIRACETAM analogs or derivatives thereof" includes any acetam compound of formula I, in racemic or isomeric form, or a pharmaceutically acceptable salts thereof, 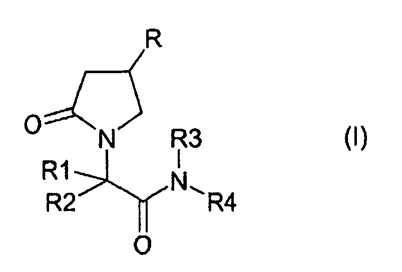 wherein
- R represents hydrogen or hydroxy;
- R1 and R2 represent independently hydrogen or an alkyl group of 1-4 carbon atoms; and
- R3 and R4 represent independently hydrogen, an alkyl group of 1-4 carbon atoms or- (CH2) n-NR5R6 wherein n is 1,2 or 3 and R5 and R6 represent independently hydrogen or an alkyl group of 1-4 carbon atoms. An example of such an acetam compound includes, but is not limited to, a compound of formula I wherein R, R1, R2, R3 and R4 are hydrogen, 2-oxopyrrolidineacetamide, known by the generic name piracetam as described in UK Patents Nos. 1,039, 113 and 1,309, 692.

As used herein, the term "LEVETIRACETAM analogs or derivatives thereof' also include optionally substituted N-alkylated 2-oxo-azepanyl derivatives. Preferably, those compounds are alkyl amides derivatives substituted on the positions 4 and/or 5 and/or 6 and/or 7 of the 2-oxo- azepanyl ring. Examples of optionally substituted N-alkylated 2-oxo-azepanyl derivatives include, but are not limited to, compounds such as those disclosed in international patent application PCT/EP02/05503 such as 2- [5- (iodomethyl)-2-oxo-1azepanyl] butanamide.

In another embodiment, the pharmaceutical composition as defined previously comprises optionally a pharmaceutically acceptable carrier.

As example of pharmaceutically acceptable carrier, the composition can comprise emulsions, microemulsions, oil-in water emulsions, anhydric lipids and water in oil emulsions, or other types of emulsions.

The pharmaceutical composition of the invention may further comprise one or more additives such as diluents, excipients, stabilizers and preservatives. Such additives are well known to one skilled in the art and are for example, described in *"*Ullmann' Encyclopedia of Industrial Chemistry, 6th Ed." (various editors, 1989-1998, Marcel Dekker); and Pharmaceutical Dosage Forms and Drug Delivery Systems (ANSEL et al., 1994, WILLIAMS & WILKINS).

Another object of the present invention concerns a method for treating photosensitive reflex epilepsy in a subject, said method comprising a step of administering an effective amount of the composition as previously described to said subject.

As used herein, a subject is an animal which has been diagnosed with photosensitive reflex epilepsy. Preferably, said subject is a human.

According to the present invention, an "effective amount" of a composition is one which is sufficient to achieve a desired biological effect, in this case at least diminishing the seizures occurring in response to Intermittent Light Stimulation. It is understood that the effective dosage will be dependent upon the age, sex, health, and weight of the recipient, kind of concurrent treatment, if any, frequency of treatment, and the nature of the effect desired. The ranges of effective doses provided below are not intended to limit the invention and represent preferred dose ranges. However, the preferred dosage can be tailored to the individual subject, as is understood and determinable by one of skill in the art, without undue experimentation. See, e.g., Berkow (1987), infra, Goodman (1990), infra, Avery (1987), infra, Ebadi, Pharmacology, Little, Brown and Co., Boston, Mass. (1985), and Katsung (1992), infra, which references and references cited therein, are entirely incorporated herein by reference.

Generally speaking, the recommended initial dose of LEVETIRACETAM is 1000 mg once daily, with gradual adjustment to a maximum of 3000 mg daily. The dosage will be adjusted according to the type of administration of the composition according to the invention.

LEVETIRACETAM is commercially available and is approved for sale in various countries including the United States of America under the brand name KEPPRA(TM) and KEPPRA XR(TM) from UCB PHARMA. It is available in four dosage forms: immediate release tablets; an oral solution; an injectable infusion; and extended release tablets.

KEPPRA is available in 250, 500, 750 and 1000 mg strengths as immediate release tablet formulations. Inactive excipients for these tablets include colloidal silicon dioxide, croscarmellose sodium, magnesium stearate, polyethylene glycol 3350, polyethylene glycol 6000, polyvinyl alcohol, talc, titanium dioxide and colouring agents.

LEVETIRACETAM is also available as KEPPRA oral solution with the strength of 100 mg/ml. Inactive excipients include ammonium glycyrrhizinate, citric acid monohydrate, glycerin, maltitol solution, methyl paraben, potassium acesulfame, propylparaben, purified water, sodium citrate dihydrate and natural and artificial flavor. LEVETIRACETAM is rapidly and almost completely absorbed after oral administration. Absorption of LEVETIRACETAM is rapid, with peak plasma concentrations occurring within about one hour following oral administration to fasted subjects. The oral bioavailability of LEVETIRACETAM tablets is 100% and the tablets and oral solution are bioequivalent in their rate and extent of absorption. Food does not affect the extent of absorption of LEVETIRACETAM, but it decreases Cmax by 20% and delays Tmax by 1.5 hours. The pharmacokinetics of LEVETIRACETAM are linear over the dose range of 500-5000 mg. Steady state is achieved after 2 days of twice-daily dosing. LEVETIRACETAM and its major metabolite are less than 10% bound to plasma proteins; clinically significant interactions with other drugs through competition for protein binding sites are therefore unlikely.

Also available is KEPPRA injectable solution of 100 mg/ml strength, wherein inactive excipients include water for injection, sodium chloride buffered at pH 5.5 using glacial acetic acid and 8.2 mg sodium acetate trihydrate.

LEVETIRACETAM is also available in extended release form as KEPPRA XR(TM) with the strength of 500 mg. Inactive ingredients include colloidal anhydrous silica, hypromellose, magnesium stearate, polyethylene glycol 6000, polyvinyl alcohol-partially hydrolyzed, titanium dioxide, macrogol/PEG 3350, and talc. Whatever dosage is used, it should be a safe and effective amount as determined by known methods, as also described herein.

One of skill in the art can also determinate according to its general knowledge the effective amount of pharmaceutical acceptable carriers to be administrated to a subject in order to achieve the desired biological effect.

### EXAMPLES

### Example 1 Identification of the mutation responsible for photosensitive reflex epilepsy

To get insights into the molecular mechanisms causing photosensitive epilepsy, the inventors realized a genome-wide scan by linkage analysis with microsatellite and SNP markers in order to identify a mutation responsible for photosensitive reflex epilepsy in chicken.

Said mutation has been identified on GGA25, a chicken microchromosome largely under-represented in the sequence assembly in a 11.6 cM interval between markers GCT1888 and 100A3M13 which have been found to comprise SV2A (synaptic vesicle glycoprotein 2A), SV2A being a very strong candidate gene, based on its potential involvement in neurotransmission.

The inventors found abnormal alternative splicing events involving SV2A exon3 in epileptic chicken. Sequencing of PCR products showed that the 106 first base pairs of SV2A exon 3 were missing in the alternatively spliced variant causing a frame shift leading to a premature termination codon 75 bp before the junction between exons 4 and 5. This could result in the synthesis of a truncated protein of 244 amino acids while the mammal wild type protein is 742 amino acids or in the degradation of the alternative mRNA through the nonsense-mediated pathway of mRNA decay. Other minor alternative transcripts were sometimes identified in the homozygous epileptic animals (data not shown).

The likely position of the mutation has been identified to be in intronic sequence, so five long-range PCR products were sequenced from two heterozygote carrier sires using the 454 technology (ROCHE). A dinucleotide mutation in the acceptor site of intron 2 was identified, at position c581-4CC>TG. This was a strong candidate mutation which may explain the abnormal splicing observed in mRNA of epileptic chicken brains. To confirm this candidate mutation, the part of SV2A intron2 containing this variant was sequenced in 185 wild type chickens from 16 different lines, 40 heterozygote carriers, and 145 epileptic chickens using the PCR primers of SEQ ID N°7 and SEQ ID N°8. All the wild type individuals carried the CC allele while carriers were heterozygous (CC/TG) and epileptics homozygous for the TG allele. The candidate mutation is located in a conserved region throughout species: the (C→G) mutation was never found in any species while the other one (C→T) is often observed. The inventors thus proposed that the real causative mutation is the C→G one. The abnormal splicing event observed seemed to be fully associated with the epileptic phenotype. However, homozygous epi mutants also express the wild type mRNA and chicken heterozygote carriers as well as mice expressing only one copy of SV2A do not display epilepsy; this suggests that half dose of the wild type transcript is sufficient to avoid seizure. SV2A knock out mice experience severe seizures and die by three weeks, showing yet that the fully absence of transcript is lethal. One hypothesis would be that the level of transcript is lower in epi homozygous mutants than in epi/+ chickens, but is still sufficient to circumvent lethality. Indeed expression level analyses of SV2A by qRT-PCR showed a genotype-dependent differential expression of the gene (p <0 .003, Kruskal-Wallis statistical test). Heterozygous carriers displayed a 2-fold down-expression when compared to wild type (0.017 +/- 0.006 versus 0.035 +/- 0.009), the remaining level of SV2A mRNA being obviously sufficient to protect animals from seizures. Epileptic chicken brains presented a very low level of SV2A mRNA (0.0019 +/- 0.0005), their SV2A expression level being 25-fold less than wild type (12.5-fold less than carriers). Northern blot analysis showing a weak signal for the normal 4.1 kb mRNA and no minor band in epi/epi was consistent with relative real-time PCR analysis. In situ hybridization analyses performed on brain cross sections confirmed that the level of SV2A transcripts is reduced in epi/epi animals compared to wild type ones.

The present data show that photosensitive epilepsy in chicken is associated with abnormal splicing events affecting the SV2A gene, which provokes a significant decrease of the gene expression in epileptic chicken. The inventors suggest that the c581-4CC> substitution in SV2A, and most probably the c581-3C>G one, constitutes the causative mutation. The present results support a gene dosage effect: the minimum threshold of SV2A quantity required to avoid seizure is reached in heterozygous animals but not in homozygous animals.

### Methods

**Animals.** Blood samples from all individuals of the experimental pedigree, born in 2002, were collected for DNA extraction. Blood samples of animals of next generations were also collected (2003: n=18; 2004: n=28; 2005: n=151; 2006: n=30; 2007: n=134; 2008: n=120; 2009: n=146).

Wild type animals used to confirm the causative mutation came from 16 different lines: two lines selected for coccidiose resistance (White Leghorn and Fayoumi origin); two divergent lines selected for meat quality (broiler lines origin); two divergent lines selected for growth curve (broiler lines origin); two divergent lines selected for residual feed intake (Rhode Island Red origin); three divergent lines selected for salmonella resistance (White Leghorn origin: experimental inbred lines from the USDA Avian Disease and Oncology Laboratory, East Lansing, MI, provided by the Institute for Animal Health (IAH), Compton, UK); the East Lansing backcross (Red Jungle Fowl and White Leghorn origin); one inbred line (Leghorn origin); one commercial broiler line; one experimental Naked Neck line (laying strain origin) and one experimental line selected for resistance to Rous sarcomas (White Leghorn origin).

**Phenotyping.** All the animals of the experimental population were tested twice for the photosensitive GRS (Genetic Reflex Seizure) also known as photosensitive reflex epilepsy, except animals born in 2005 that were tested only once. The first phenotyping test took place at birth and the second one at one week (2009), three weeks (2002), six weeks (2007 and 2008) or eight weeks (2003-2006) of age through Intermittent Light Stimulation at 14 flashes per second, the most effective epileptogenic frequency for the chickens. Animals were classified as epileptic when they displayed a seizure during at least one of the two tests.

**Genotyping.** PCR amplifications and markers genotyping were performed through classical methods as SSCP (Single-Strand Conformation Polymorphism) analysis on an ABI 3100 sequencer (APPLIED BIOSYSTEMS), as described in APPLIED BIOSYSTEMS Publication 116AP01-02 or by sequencing, and analyzed on an ABI 3730 sequencer (Applied Biosystems). Linkage analysis was performed using CriMap version 2.4 software. The build option was used to order markers within the linkage group. The flips option was used to examine the order of the different loci by inverting every two or three loci.

### Sequencing.

Sanger technology. PCR fragments were amplified on an ABI 9700 thermocycler (Applied Biosystems) from two wild type, two carriers and two epileptic chicken cDNA using GC-rich PCR system (Roche Applied Science). Fragments were purified using 0.5U of SAP (Shrimp Alkalin Phosphatase, Promega) and 0.5U of exonuclease I (NE biolabs). Fragments were sequenced using Big Dye terminator v3.1 kit (Applied Biosystems), and analyzed on an ABI 3730 or an ABI 3100 sequencers (Applied Biosystems).

454 technology. Long-range PCR fragments were amplified on an ABI 9700 thermocycler (APPLIED BIOSYSTEMS) from the two heterozygous sires using Long PCR Enzyme Mix (Fermentas). Fragments were purified, pooled at equal concentrations and mixed with PCR fragments from other species (data not shown) to make the best use of a half-sequencing run.

Fragments were sequenced using the ROCHE 454 Life Sciences Genome Sequencer FLX following the manufacturer's instructions (454 Life Science, ROCHE). The shotgun library was prepared with 1 µg genomic DNA using the "Titanium General Library Preparation Kit". Nebulized, purified, and adaptors-linked DNA fragments were amplified using the "GS FLX Titanium LV emPCR Kit". Sequencing on the Genome Sequencer FLX was performed using the "GS FLX Sequencing Kit Titanium Reagents XLR70". A total of 245,625 reads with an average length of 340 bases were obtained.

Chicken sequences were extracted through reads alignment with the available Sanger sequence of the region and contig building using the AMOS comparative assembler. SNP were filtered using the following rules: availability of at least 20 reads of the region, minimum allele frequency higher than 15%, homopolymer SNP excluded.

**RNA extraction.** Total RNA was extracted from adult and embryo (E17) chicken brains according to classical techniques.

**Relative real-time** PCR. cDNA were obtained in a 20 µl reaction volume using 2 µg of total RNA, 1 µM of polydT primer (ROCHE), 200 U of superscript II reverse transcriptase (INVITROGEN), 40 U of RNasin (PROMEGA) and 0.04 µM of dNTP. Expression of SV2A was analyzed by the relative expression method. ACTB (β-actin) gene expression was used to normalize the amount of each investigated transcript. PCR was performed in 10 µl reaction volumes using LightCycler®480 SYBR Green I Master (ROCHE) and 3 µM each of forward and reverse primers. The PCR reactions were done using a LightCycler 480 instrument (ROCHE). All samples were analyzed in duplicate.

**Northern blot analysis.** RNA was electrophoretically separated on a denaturing formaldehyde agarose gel, transferred to a nylon membrane (MILLIPORE) and immobilized by UV irradiation. ³³P-labeled probes were generated from two PCR products of SV2A cDNA and one PCR product of GAPDH (reference gene) using "prime-a-gene" protocol (PROMEGA), and were purified with microspin G50 protocol (GE HEALTHCARE). Hybridization buffer was composed of SSC 5X, Denhardt's 5X and SDS 0.5%; washing buffer was composed of SSC 0.2X and SDS 0.1 %. Nylon membranes were pre-hybridized during 2h at 65°C with hybridization buffer and 10 µg/ml sonicated salmon sperm DNA, and hybridized over-night at 65°C with the addition of 33P-labeled probes (2 000 000 CPM/ml of hybridization buffer). Nylon membranes were washed twice (15 minutes and 5 minutes) and exposed 24h for GAPDH probe and 7 days for SV2A probes. Nylon membranes screen was scanned with Fujibas 5000 instrument, and results were analyzed using ArrayGauge V1.3.S.

**In situ hybridization analysis**. In situ hybridization was performed on vibratome (LEICA VT 1000S VIBRATING BLADE MICROTOME) cross sections of the brain obtained from 17-day old embryos. Nonradioactive RNA in situ hybridizations were performed on 50 µm brain sections using digoxigenin-UTP (ROCHE) with a labeled RNA probe prepared from PCR product of 680 pb of SV2A cDNA.

### Example 2 Test of the LEVETIRACETAM on the animal model

The inventors have tested the antiepileptic effect of LEVETIRACETAM (Keppra®, UCB PHARMA) in the chicken model according to the invention.

71 Fepi 3-5 months old and identified for ILS seizures at the birth were tested with LEVETIRACETAM.

During 21 days, they were exposed to ILS (3 minutes) once a day.

Behavioural activities were observed and time recorded for each phase of the paroxysmal manifestation during controlled intermittent light stimulation:
- T0: ILS starts
- T1 (time in seconds): neck myoclonus starts
- T2 (time in seconds): convulsive phase starts
- T3 (time in seconds): convulsive phase ends

The first day they were tested for control and all had ILS induced seizures.

The following 10 days all the animals received an intraperitoneal injection, 18 animals with 200 mg/kg of LEVETIRACETAM, 17 with 100 mg/kg, and 18 with 50 mg/kg, while 18 control animals received a placebo injection. Each treatment category (200, 100, 50, 0) included equal proportions of males and females.

During the last 10 days, none of the animals was treated.

The influence of the treatment on the stimulus locked myoclonus and generalized convulsions of the photosensitive GRS was evaluated using generalized linear models implemented in the function glm of the software R, version 2.9.0. The number of seizures for each animal over a given phase of the experiment (with treatment and following treatment) was modeled using a binomial distribution, and linked to factors using the logit function. The delay for seizure initiation, the duration of the myoclonus and the duration of the convulsions were modeled using gamma distributions, and linked to the factors using the identity function. Sex was included as an additive co-factor in the analyses but not significant differences were found.

The results showed reduced seizure number (odds ratio 0.03, p-value 9 e-07), delayed myoclonus appearance (from 22.8 s to 25.1 s on average, p-value 0.04) and reduced seizure duration (from 16.2 s to 10.7 s on average, p-value 1 e-08) in treated (50, 100 or 2000 mg/Kg) versus placebo homozygous, the effect partially persisting few days after the injection period. These findings indicate that LEVETIRACETAM has an anticonvulsant effect in the chicken model of the invention, as in human and mice epileptic models.

### Example 3 Diagnosis of photosensitive reflex epilepsy

The invention provides a method for diagnosing photosensitive reflex epilepsy in a subject.

Firstly, a biological sample from the subject to be diagnosed with or without photosensitive reflex epilepsy is removed. Said biological sample can be provided from various origins depending on whether the method of diagnosis will detect the presence of a mutation in the SV2A gene or will determine the expression level of the SV2A gene.

Secondly, the biological sample from the subject to be tested is used for detecting the presence of a mutation in the SV2A gene leading to an underexpression or absence of expression of said gene, and/or determining the expression level of the SV2A gene.

In the case of the detection of a mutation in the SV2A gene, said biological sample will preferably contain cells including genomic DNA and/or RNA.

In the case of the determination of the expression level of the SV2A gene, the biological sample will contain neuronal or endocrinal cells, which are the only cells in which the SV2A proteins are produced.

The detection of the mutation can be assessed by using typical techniques for detecting the presence of a mutation as described previously.

The determination of the expression level of a gene can be assessed by methods as explained previously.

The determination of the expression level of the SV2A gene can also be assessed by analyzing the expression of the SV2A protein translated from said gene by using analysis as described here above, especially with the use of an antibody directed against SV2A.

The results of the present diagnostic test enable to determine whether or not a patient is subject to photosensitive reflex epilepsy.

## Claims

1. A method for diagnosing photosensitive reflex epilepsy in a subject, which comprises the steps of analyzing a biological sample from said subject by:
i) Detecting the presence of a mutation in the Synaptic Vesicle protein 2 A (SV2A) gene leading to an underexpression or absence of expression of said gene, and/or
ii) Determining the expression level of the SV2A gene,
wherein said mutation and/or expression level of the SV2A gene is indicative of photosensitive reflex epilepsy in said subject.

2. The method according to claim 1, wherein said method comprises the step of detecting the presence of a mutation in the SV2A gene leading to the underexpression or absence of expression of said gene.

3. The method according to claim 1, wherein said method comprises the step of analyzing the expression of the Synaptic Vesicle protein 2 A (SV2A) gene.

4. The method according to claim 3, wherein said analyzing step is assessed by analyzing the expression of mRNA transcript or mRNA precursors of said gene in a biological sample containing cells including RNA.

5. The method according to claim 3, wherein said analyzing step is assessed by analyzing the expression of the SV2A protein translated from said gene.

6. A method of predicting the efficiency of a treatment of a subject diagnosed with photosensitive reflex epilepsy with the method according to any one of claims 1 to 5, which comprises the step of analyzing a biological sample from said subject by determining the expression level of the SV2A gene, wherein the expression level of the SV2A gene is indicative of the efficiency of said treatment on said subject.

7. A kit for diagnosing photosensitive reflex epilepsy in a subject or for predicting the efficiency of a treatment of a subject diagnosed with photosensitive reflex epilepsy, wherein said kit comprises at least one nucleic acid probe or oligonucleotide or at least one antibody, which can be used in a method according to any one of claims 1 to 6, for detecting the presence of a mutation in the SV2A gene and/or analyzing the expression of the SV2A gene.

8. Use of the kit according to claim 7 for diagnosing photosensitive reflex epilepsy in a subject.

9. Use of the kit according to claim 7 for predicting the efficiency of a treatment of a subject diagnosed with photosensitive reflex epilepsy.

10. A method for identifying a compound useful in the treatment of photosensitive reflex epilepsy, the method comprising the steps of
(a) Administering said compound to a non-human photosensitive epileptic animal, preferably a chicken, presenting a mutation of the Synaptic Vesicle protein 2 A (SV2A) gene leading to the underexpression of said gene;
(b) Measuring the responsiveness of said non-human treated animal to Intermittent Light Stimulation (ILS);
(c) Comparing the responsiveness to intermittent light stimulation of said treated animal with the responsiveness of a non-human non treated animal to ILS;
(d) Selecting a compound for which said treated animal presents an absent or diminished photosensitive reflex epileptic phenotype compared to a non treated animal.

11. The method according to claim 10, wherein said mutation is located in the acceptor site of the second intron of the SV2Agene.

12. The method according to claim 11, wherein said mutation is detected with PCR primers of SEQ ID N°7 and SEQ ID N°8.

13. The method according to any one of claims 10 to 12, wherein said method comprises a former step of selecting a non-human photosensitive epileptic animal having a mutation in the SV2A gene leading to the underexpression of said gene.

14. The method according to any one of claims 10 to 12, wherein said method comprises a former step of determining the expression level of the SVA gene in the non-human animals used according to the invention.

15. Use of a non-human photosensitive epileptic animal presenting a mutation of the Synaptic Vesicle protein 2 A (SV2A) gene leading to the underexpression or absence of expression of said gene for identifying compounds useful for the treatment of photosensitive reflex epilepsy.


SEQUENCE LISTING

<110> INSTITUT NATIONAL DE RECHERCHE AGRONOMIQUE
PITEL, Frédérique

<120> METHOD FOR IDENTIFYING COMPOUNDS USEFUL IN THE TREATMENT OF
PHOTOSENSITIVE REFLEX EPILEPSY

<130> INR-B-0002 EP1

<160> 9

<170> PatentIn version 3.5

<210> 1
<211> 4431
<212> DNA
<213> Homo sapiens

<400> 1
aggcggggct ggagtggtgg aaggggggtg gcaggtctgc attgccgctt ccctggtgcc 60
gggagcagtc gccgctgccg cctccgcccg cggccgggac ccccgtcctc gcccgggact 120
ccttacccgg ggaacctaga ccaggtctcc agaggcttgt ggaagagaag caggcgaccc 180
ttcctgagtt atcctggctt agcctcccaa tctggctccc cttccccttc ccattcccct 240
gctccccctg tcccttcccc atccacccaa ctgaactggg tataggtcaa agctcctctc 300
tttccttttc cttcctaggc actcattggc taggacctgt ttgctctttt ttttgtgccc 360
agagatactg gaacacgctt catctaagta actgtgggga ggggtctttt tgactctaca 420
agtccttgag caaaaagctg aaaaagaagc aggaggtgga gaagacccag tgaagtgccc 480
caagccccat catggaagag ggcttccgag accgggcagc tttcatccgt ggggccaaag 540
acattgctaa ggaagtcaaa aagcatgcgg ccaagaaggt ggtgaagggc ctggacagag 600
tccaggacga atattcccga agatcgtact cccgctttga ggaggaggat gatgatgatg 660
acttccctgc tcccagtgat ggttattacc gaggagaagg gacccaggat gaggaggaag 720
gtggtgcatc cagtgatgct actgagggcc atgacgagga tgatgagatc tatgaagggg 780
aatatcaggg cattccccgg gcagagtctg ggggcaaagg cgagcggatg gcagatgggg 840
cgcccctggc tggagtaagg gggggcttga gtgatgggga gggtccccct gggggccggg 900
gggaggcaca acgacggaaa gaacgagaag aactggccca acagtatgaa gccatcctac 960
gggagtgtgg ccacggccgc ttccagtgga cactgtattt tgtgcttggt ctggcgctga 1020
tggctgacgg tgtggaggtc tttgtggtgg gcttcgtgct gcccagcgct gagaaagaca 1080
tgtgcctgtc cgactccaac aaaggcatgc taggcctcat cgtctacctg ggcatgatgg 1140
tgggagcctt cctctgggga ggtctggctg accggctggg tcggaggcag tgtctgctca 1200
tctcgctctc agtcaacagc gtcttcgcct tcttctcatc ttttgtccag ggttacggca 1260
ctttcctctt ctgccgccta ctttctgggg ttgggattgg agggtccatc cccattgtct 1320
tctcctattt ctccgagttt ctggcccagg agaaacgagg ggagcatttg agctggctct 1380
gcatgttttg gatgattggt ggcgtgtacg cagctgctat ggcctgggcc atcatccccc 1440

actatgggtg gagttttcag atgggttctg cctaccagtt ccacagctgg agggtcttcg 1500
tcctcgtctg cgcctttcct tctgtgtttg ccattggggc tctgaccacg cagcctgaga 1560
gcccccgttt cttcctagag aatggaaagc atgatgaggc ctggatggtg ctgaagcagg 1620
tccatgatac caacatgcga gccaaaggac atcctgagcg agtgttctca gtaacccaca 1680
ttaagacgat tcatcaggag gatgaattga ttgagatcca gtcggacaca gggacctggt 1740
accagcgctg gggggtccgg gccttgagcc taggggggca ggtttggggg aattttctct 1800
cctgttttgg tcccgaatat cggcgcatca ctctgatgat gatgggtgtg tggttcacca 1860
tgtcattcag ctactatggc ctgaccgtct ggtttcctga catgatccgc catctccagg 1920
cagtggacta cgcatcccgc accaaagtgt tccccgggga gcgcgtagag catgtaactt 1980
ttaacttcac gttggagaat cagatccacc gaggcgggca gtacttcaat gacaagttca 2040
ttgggctgcg gctcaagtca gtgtcctttg aggattccct gtttgaagag tgttattttg 2100
aggatgtcac atccagcaac acgtttttcc gcaactgcac attcatcaac actgtgttct 2160
ataacactga cctgttcgag tacaagtttg tgaacagccg tctgataaac agtacattcc 2220
tgcacaacaa ggagggctgc ccgctagacg tgacagggac gggcgaaggt gcctacatgg 2280
tatactttgt gagcttcctg ggacactggc cagtgcttcc tgggaatatc gtgtctgccc 2340
tgctcatgga caagatcggc aggctcagaa tgcttgctgg ctccagcgtg atgtcctgtg 2400
tctcctgctt cttcctgtct ttgggaacag tgagtcggcc atgatcgctc tgctctgccc 2460
tttttggcgg ggtcagcatt gcatcctgga atgcgctgga cgtgttgact gttgaactct 2520
acccctcaga caagaggacc acagcttttg gcttcctgaa tgccctgtgt aagctggcag 2580
ctgtgctggg gatcagcatc ttcacatcct tcgtgggaat caccaaggct gcacccatcc 2640
tctttgcctc agctgccctt gcccttggca gctctctggc cctgaagctg cctgagaccc 2700
gggggcaggt gctgcagtga aggggtctct agggctttgg gattggcagg cacactgtga 2760
gaccaacaac tccttccttc ccctccctgc cctgccatcc tgacctccag agccctcact 2820
ccccactccc cgtgtttggt gtcttagctg tgtgtgcgtg tgcgtgtgca tgtgtgtaaa 2880
ccccgtgggc agggactaca gggaaggctc cttcatccca gttttgagat gaagctgtac 2940
tccccatttc ccactgccct tgactttgca caagagaagg ctgagcccca tccttctccc 3000
cctgttagag aggggccctt gcttccctgt tccaggggtt ccagaatagg cttcctgcct 3060
tccccatcat tccctctgcc taggccctgg tgaaaccaca ggtatgcaat tatgctaggg 3120
gctggggctc tggtgtagac catggaccaa aagaacttct tagagtctga agagtgggcc 3180
tcgggtgccc tctcacatct cctgttggat gctgggggag aagcaataaa cctcagccct 3240
ctggcctcca ctttcctctc aatttgggct gcaaatatga agcctgaatt ttatgaaatt 3300
agctttctga ttcttattta ttaatagatt aagttctgag gcagctccgc aggactgtgt 3360
gtgaatgtgt atgtatactt acatatgtgt gtgcatgtgc catggggcgg ggggtatcac 3420
tatactgtcc tcaaatataa gccaagggta atttcagcgg atgcacacac aaccctgcct 3480

cccacagttc ctcccctaat ctggtttctg tgttgagcct gggatggagg agccctaggc 3540
cagcctggga taagagtccc acagtctagg gagatctgag ggcatccgac aaggcccatc 3600
tccttccctc ctcaagaagc agaggcctcc tctggagtga gaggctccac ccactacagc 3660
acaggcggga atagcacagc tgccctccca tgctccctac ctgtcccctc acagggaggg 3720
gagcagggga gggaaagaaa ccaggcatct ggtcaaacca gcagatcaaa aagcacaaag 3780
agctggggca gaggcaggaa gcaggggccc tcctggcagc tcctctgagt ggggagaggt 3840
tgggcagtga gtgagggacc cctaatgcag ggactagaag cctcagtttc cccattttac 3900
ccttccacac aatagcctct gtaggttagg ctgccccatc ccaccctact ctgtgtggct 3960
gctttctttg gtgccctccc ctcaccccac tgtagctgtg acgtgttgta gtttttagat 4020
gtttgtaaaa tgtttaaaaa aatgttaaaa ggaaaaaagt gaaaataaca aaaaagaaaa 4080
tcaaaattca ccttcgtcat gctgcgtcca gtgccccaac cctgtggtca ctctccccat 4140
tttgtaacac tgtaccaggt ggtgactgtt taactctttg gtgtctgtgc tcaaaagact 4200
gccttctcca gtgcccagtg tatgagtgtg tgccctgtgc ccttgtccct cactccccac 4260
atgctggacg tagccctctt cctcgcaccc ctgggaggga cccatccatc tcccttgctc 4320
tcctggggaa ccctaaaccc aactctgttg atgtgaaaaa tgcagtgaaa aatattgacg 4380
aaaaataaaa cggaaacaaa tcctcaaaat acaaaaaaaa aaaaaaaaaa a 4431

<210> 2
<211> 742
<212> PRT
<213> Homo sapiens

<400> 2

Met Glu Glu Gly Phe Arg Asp Arg Ala Ala Phe Ile Arg Gly Ala Lys
1               5                   10                  15

Asp Ile Ala Lys Glu Val Lys Lys His Ala Ala Lys Lys Val Val Lys
            20                  25                  30

Gly Leu Asp Arg Val Gln Asp Glu Tyr Ser Arg Arg Ser Tyr Ser Arg
        35                  40                  45

Phe Glu Glu Glu Asp Asp Asp Asp Asp Phe Pro Ala Pro Ser Asp Gly
    50                  55                  60

Tyr Tyr Arg Gly Glu Gly Thr Gln Asp Glu Glu Glu Gly Gly Ala Ser
65                  70                  75                  80

Ser Asp Ala Thr Glu Gly His Asp Glu Asp Asp Glu Ile Tyr Glu Gly
                85                  90                  95

Glu Tyr Gln Gly Ile Pro Arg Ala Glu Ser Gly Gly Lys Gly Glu Arg
            100                 105                 110

Met Ala Asp Gly Ala Pro Leu Ala Gly Val Arg Gly Gly Leu Ser Asp
        115                 120                 125

Gly Glu Gly Pro Pro Gly Gly Arg Gly Glu Ala Gln Arg Arg Lys Glu
    130                 135                 140

Arg Glu Glu Leu Ala Gln Gln Tyr Glu Ala Ile Leu Arg Glu Cys Gly
145                 150                 155                 160

His Gly Arg Phe Gln Trp Thr Leu Tyr Phe Val Leu Gly Leu Ala Leu
                165                 170                 175

Met Ala Asp Gly Val Glu Val Phe Val Val Gly Phe Val Leu Pro Ser
            180                 185                 190

Ala Glu Lys Asp Met Cys Leu Ser Asp Ser Asn Lys Gly Met Leu Gly
        195                 200                 205

Leu Ile Val Tyr Leu Gly Met Met Val Gly Ala Phe Leu Trp Gly Gly
    210                 215                 220

Leu Ala Asp Arg Leu Gly Arg Arg Gln Cys Leu Leu Ile Ser Leu Ser
225                 230                 235                 240

Val Asn Ser Val Phe Ala Phe Phe Ser Ser Phe Val Gln Gly Tyr Gly
                245                 250                 255

Thr Phe Leu Phe Cys Arg Leu Leu Ser Gly Val Gly Ile Gly Gly Ser
            260                 265                 270

Ile Pro Ile Val Phe Ser Tyr Phe Ser Glu Phe Leu Ala Gln Glu Lys
        275                 280                 285

Arg Gly Glu His Leu Ser Trp Leu Cys Met Phe Trp Met Ile Gly Gly
    290                 295                 300

Val Tyr Ala Ala Ala Met Ala Trp Ala Ile Ile Pro His Tyr Gly Trp
305                 310                 315                 320

Ser Phe Gln Met Gly Ser Ala Tyr Gln Phe His Ser Trp Arg Val Phe
                325                 330                 335

Val Leu Val Cys Ala Phe Pro Ser Val Phe Ala Ile Gly Ala Leu Thr
            340                 345                 350

Thr Gln Pro Glu Ser Pro Arg Phe Phe Leu Glu Asn Gly Lys His Asp
        355                 360                 365

Glu Ala Trp Met Val Leu Lys Gln Val His Asp Thr Asn Met Arg Ala
    370                 375                 380

Lys Gly His Pro Glu Arg Val Phe Ser Val Thr His Ile Lys Thr Ile
385                 390                 395                 400

His Gln Glu Asp Glu Leu Ile Glu Ile Gln Ser Asp Thr Gly Thr Trp
                405                 410                 415

Tyr Gln Arg Trp Gly Val Arg Ala Leu Ser Leu Gly Gly Gln Val Trp
            420                 425                 430

Gly Asn Phe Leu Ser Cys Phe Gly Pro Glu Tyr Arg Arg Ile Thr Leu
        435                 440                 445

Met Met Met Gly Val Trp Phe Thr Met Ser Phe Ser Tyr Tyr Gly Leu
    450                 455                 460

Thr Val Trp Phe Pro Asp Met Ile Arg His Leu Gln Ala Val Asp Tyr
465                 470                 475                 480

Ala Ser Arg Thr Lys Val Phe Pro Gly Glu Arg Val Glu His Val Thr
                485                 490                 495

Phe Asn Phe Thr Leu Glu Asn Gln Ile His Arg Gly Gly Gln Tyr Phe
            500                 505                 510

Asn Asp Lys Phe Ile Gly Leu Arg Leu Lys Ser Val Ser Phe Glu Asp
        515                 520                 525

Ser Leu Phe Glu Glu Cys Tyr Phe Glu Asp Val Thr Ser Ser Asn Thr
    530                 535                 540

Phe Phe Arg Asn Cys Thr Phe Ile Asn Thr Val Phe Tyr Asn Thr Asp
545                 550                 555                 560

Leu Phe Glu Tyr Lys Phe Val Asn Ser Arg Leu Ile Asn Ser Thr Phe
                565                 570                 575

Leu His Asn Lys Glu Gly Cys Pro Leu Asp Val Thr Gly Thr Gly Glu
            580                 585                 590

Gly Ala Tyr Met Val Tyr Phe Val Ser Phe Leu Gly Thr Leu Ala Val
        595                 600                 605

Leu Pro Gly Asn Ile Val Ser Ala Leu Leu Met Asp Lys Ile Gly Arg
    610                 615                 620

Leu Arg Met Leu Ala Gly Ser Ser Val Met Ser Cys Val Ser Cys Phe
625                 630                 635                 640

Phe Leu Ser Phe Gly Asn Ser Glu Ser Ala Met Ile Ala Leu Leu Cys
                645                 650                 655

Leu Phe Gly Gly Val Ser Ile Ala Ser Trp Asn Ala Leu Asp Val Leu
            660                 665                 670

Thr Val Glu Leu Tyr Pro Ser Asp Lys Arg Thr Thr Ala Phe Gly Phe
        675                 680                 685

Leu Asn Ala Leu Cys Lys Leu Ala Ala Val Leu Gly Ile Ser Ile Phe
    690                 695                 700

Thr Ser Phe Val Gly Ile Thr Lys Ala Ala Pro Ile Leu Phe Ala Ser
705                 710                 715                 720

Ala Ala Leu Ala Leu Gly Ser Ser Leu Ala Leu Lys Leu Pro Glu Thr
                725                 730                 735

Arg Gly Gln Val Leu Gln
            740

<210>  3
<211>  51
<212>  DNA
<213>  homo sapiens

<220>
<221>  n
<222>  (26)..(26)
<223>  n is T or A

<220>
<221>  misc_feature
<222>  (26)..(26)
<223>  n is a, c, g, or t

<400>  3
gtcctgtgtc tcctgcttct tcctgncttt tgggaacagt gagtcggcca t                51

<210>  4
<211>  61
<212>  DNA
<213>  homo sapiens

<220>
<221>  n
<222>  (31)..(31)
<223>  n is A or G

<220>
<221>  misc_feature
<222>  (31)..(31)
<223>  n is a, c, g, or t

<400>  4
tcaaacaggg aatcctcaaa ggacactgac ntgagccgca gcccaatgaa cctgtaaggc        60

c                                                                        61

<210>  5
<211>  61

<212>  DNA
<213>  homo sapiens


<220>
<221>  n
<222>  (31)..(31)
<223>  n is C or T

<220>
<221>  misc_feature
<222>  (31)..(31)
<223>  n is a, c, g, or t

<400>  5
acacacccat catcatcaga gtgatgcgcc natattcggg accaaaacag gagagaaaat      60

t                                                                      61


<210>  6
<211>  61
<212>  DNA
<213>  homo sapiens


<220>
<221>  n
<222>  (31)..(31)
<223>  n is G or T

<220>
<221>  misc_feature
<222>  (31)..(31)
<223>  n is a, c, g, or t

<400>  6
cactgcctcc gacccagccg gtcagccaga nctccccaga ggaaggctcc caccatcatg      60

c                                                                      61


<210>  7
<211>  17
<212>  DNA
<213>  artificial sequence

<220>
<223>  Primer

<400>  7
tgtccctgct acccggg                                                     17


<210>  8
<211>  21
<212>  DNA
<213>  artificial sequence

<220>
<223>  Primer

<400>  8
cagaacagga aggtgccgta g                                                21


<210>  9
<211>  16522

<212>  DNA
<213>  gallus gallus


<220>
<221>  misc_feature
<222>  (6858)..(6867)
<223>  n is a, c, g, or t

<220>
<221>  misc_feature
<222>  (11668)..(11677)
<223>  n is a, c, g, or t

<220>
<221>  misc_feature
<222>  (15070)..(15079)
<223>  n is a, c, g, or t

<400>  9
   aatgggagct caattctttc ccagctccat cctttcctgt gctggtcccg ctgcgttgcc      60
   agccccagtt ctgtgcctcc aacccctgct tgtgtggtct gagagtctct gctgccccag     120
   tggcagtctg gggactctcc aaccctcact gtgcccttgg cagacttcag cctcgcgtct     180
   gccacatcgt tcctcatcct ctgcttgaag gaggtcgggg tcctacaggg ctctgctgat     240
   ggctctgggg ctgagctcct ggagctgagg ctggctcggc tcgtggcatg tgggaaagca     300
   ttgccctgta tgtcctcccc cgccacccaa aaggcatcag agggctctgt gccgtatcct     360
   gatggagggc tgagcctgac cagtccaggc ggagaaaatc tgatctgaag caatcgaaat     420
   tcacccctga ggaggatgtg actcagatct gcctggctgt gcttccatct gcccgcggcc     480
   gtggtgggag cagcgggaga atctgccaga gcggttcgga acaccgtgaa acccttttgc     540
   tccgcaccaa acccccggca cggggccggg acccacagct caccccccac agatccatcc     600
   ccagagcaac cccagcactg gagccccgac cccggggaag ctcccggcac ccgacggagc     660
   gtcgcgtcgc atcccttcgg ctctgctata accaaaggac ctgagacagc cccccctcac     720
   cggcaccccc ttcccccccc gccccgctgt agcacagcct ccccatcgcc tcccttctca     780
   ggctgtcgca atggactctc ccagcgctcg gtgccgtgtg gcggaggctg cagcgctgac     840
   gccgggctgg tacccgcggg ctgctcctgc tggatcccca cgagcatcct tcctcctcgg     900
   agcatccttc ctcctcgcag ccggcgctgg agcggaccac gcgctgacac aggtaaggag     960
   aggaggatgc ctctataaag cgttcccccc cctcctctcc catctccttt taattagcat    1020
   cctcggggtt gctcgctgac ggatggggag ttcgccttct ccttcccatt gctgtggcca    1080
   ttttgtcctg aaaatccttg cctccacctc gctctgcctt tctctgcgtg gtcgggagcc    1140
   cttcgttacc gctcagcgct gcgcaccgca gggatcgcgt tatggggatg gatagcgatc    1200
   ccggcgggtt ttggggaggg ggagtgccac agctccccgg ggtgaggggt ggggggtgac    1260
   cttgggcacc ggggagtgcc aactcttccc catgtcgcca tgttcatggt gttctgcggt    1320
   agggctgtgg ggggaccctg atatgggaaa ccccgggggt ttggggcacg cagcaatgtg    1380
   gggccgcgcc aatcgtgtgc tcagagggag gaagggggtc cagagcccca taaccttgca    1440
   cagggccggc agtggtggcc ggggctgacg tgagggatgg gatgtctgag cctttgtgca 1500
   gccaggagag catttttggg aacccccacc tccactcccc atcttgttag gctgcgatct 1560
   gtgccgtttc tgccttcctt gggaaagcag aacccccatc ctaaaaggat ccgctgtcgg 1620
   tgtgtggtgg cccgatgctg tgtggccccc agctgtgacc ttgccccccg caggtgctcg 1680
   tacccccccc ccccccccga gctccccact ccctttgcct gatccctatt gccaaaatgc 1740
   gtcacagccc cgtggaagcg gccctgcacc agatgacctt ggtggtggtg aaggccgcag 1800
   attgactcct tccagggctg ctgggattcc acccgaggag ggcagcgggt gatgaggatt 1860
   tgatgcccgg ggatggaagg tttggggagg ggggagagga gggggggttg gatggagggg 1920
   ggggggggtg ttgtgccgca gcgccagcac cgcactgtcc ctgggtgtct gcgcagagcc 1980
   ggcgtgggtt gggtgccccc ccccggggga gcgctgccga cagccccgga aaatttggaa 2040
   aggccaaata tagctctacg ctgcgcccca gccctgctct tatggtgggg ggggtcggtg 2100
   gggggagaca cgctgtgacg gctgcgtccc tccctgcggc acaactcctg agtcgcggcc 2160
   actgctgcct ggcgggtttg tgctgccaga agtaaaaaaa acccaatttg gggggggggg 2220
   gggggggagg gggttcatgc tgggtcagca gtgcccatcc ccggctgggg ggccgtgggg 2280
   tcctgtccct ggcacgcgtt gtgccgggct ctgcaagggg caatgcaggg tgatgcccat 2340
   ggccaggtgg ggctgaggga ttggacaccc caggatgggg gcccttgggg tgtatccgga 2400
   cacccccttt cctcccaaaa ctcagatgca gcggtggtca gagcgtgccc ggctctgcct 2460
   caggctctca gctttggcca ggggctctga tggaacgcac ctgcagccag tctccttctg 2520
   gaagcctccc tggggtgggc atgcgtggtc tccatgccca ctctgcaccc acggccacct 2580
   ctgggcagct ggattggagc tgggcgagcc tggacctccc ctgagggtcc cccgacccaa 2640
   acctcacttt gctccctgcc tgtggatggc caccgatgca gagcactggt gctgccctgg 2700
   gaggtttggg gacccccgtg tccccggttc cttcactgtc cctgtggggc acggtgacag 2760
   taccgctcgg ttcccgaagc actggcgctt tgtgggggcc tgctggctgt cccagttcct 2820
   ggtggtgacc ccacagtgtc ttcctcatcc tctgtccctc acggcgctga cccacagggc 2880
   tggctgtccc caggccctcg ctgtcatcgg cgccctgcca ccctgggggc cgctgcgtgc 2940
   cctgctgggg tcagcatccc ccgggggtcc cgacccggcg ctgcgacccc tcagcccgga 3000
   ggtggtggga gcgaagggcg ggctcctgcg tggccacgcc ccccgaagct cattggctga 3060
   acggcggggc cgtgcccctc cagccccatt gatgcaggtg gggtggccac gcccccgtaa 3120
   gctcattggt tcgtggccca gcctcgtctt cccagcccca ttgagagggc ggtggggcct 3180
   tatttctcca tgggttgggc ggcctggtag ggggctgcgg tggggatagg ggcttccccg 3240
   cggcctgggg accttggctt gtcggtgggg agctgtgggt ccgtgctggg gatcccccat 3300
   gagactctgg gggcacagat aggtctggag atgtggcccc ctcggcatcc tgctgagatg 3360
   cagatgcccc accaggatgt ggggagggct ctggagctgc tgtgtccctc catacctcac 3420
   cttcccaagt cccgattcct gtgtgcctcc atagaatatc ttctcccatt ccccatatcc 3480
   cacgtgtctc tccagatcct atctgctcac gttccccgca cccgtgggcg cctgcccctg 3540
   cgctgggctc tcgctatgcc atgtccctgc agccttgctg tggggcagag gtgcccccaa 3600
   atcctccagt tgccatccaa gtgctgcacg ctgagcctcg tgctggacgt gcagaccctg 3660
   aggcgaggtg ggacacgatg acgtccccac cctaacacgg ccactgtcgt gtctgggtcc 3720
   ccaggtgtgg gccccgcgga tgggtagcgc tgctgcccaa ccctccgcat ccgaccgcgg 3780
   cccccaggcg tgagggctca gccccgacac agagaccgcc cccaaaktcc cacgccgcyg 3840
   ccccaaccca acgtcgccat ggatgagagc ttccgagacc ggacggcgtt catccgcggc 3900
   gccaaagaca tcgccaagga ggtgaagaag cacgcggcca agaaggtgag caaaggcatg 3960
   gaccgcgtgc aggacgagta caccaagcgc tcctacagcc gcttcgagga ggaggacgac 4020
   gacgaggact acgcgccgca ggacggctac taccggggcg gtgagggggc ggccgaggag 4080
   gagggggctt ccagcgatgc caccgaaggg cacgacgagg aggacgagat ctacgagggc 4140
   gagtaccagg gcatcccgcg gcaggactcg ctgaagggcg gcgagaggct ggggggcgag 4200
   gtgacccccg gggccttcga tgacagcgag gggcagcggc gcaaggacaa ggaggagctg 4260
   gcgcagcagt acgagctcat cctgcaggag tgcggccacg ggcgcttcca gtggagcctc 4320
   tacttcgtgc tgggactggc gctgatggct gacggcgtgg aggtgttcgt ggtgggcttc 4380
   gtgctgccca gcgccgagaa ggacatgtgc ctctccgact ccaacaaggg catgctgggt 4440
   gagtggggag aggaggtggg ctgggggggg gagggacgcg ggtgtgggga ggtgggctgg 4500
   ggtgtggggg ggatatgtgg gctgtggggg tcgggaggag gtggggtatg gagggacgtg 4560
   tgggatgtag gggcgggaga agttacagtg gggagggaca caggcgtggg gacgtgggaa 4620
   gatggaggga cgtgtgggat gtgggaacag gagcagctgg ggggcattgg gggacacagg 4680
   cgaggagatg tggaggtggg ggatatgatg gagcgcaggt ttgggggtga gggcggagag 4740
   gatatggggg gacgtgtggg atatggggga cgtgggcaga cacagaatgg agggacacag 4800
   gcgtgtggat acgggcaggt ggggtatgga gacatgtggg gtatggggga cgtgggcagg 4860
   cagagtatgg gatgtgggaa gacgggatat gaggggactc atgggatatg ggggacatgg 4920
   acatacaggg catggaggga tggagatatg ggggacatgg gtatataggg tgtggaggga 4980
   tggagatatg ggggacatgg gtwtataggg tggggaggga tggagatatg ggggacatgg 5040
   gtatataggg tgtggaggga tggagatatg ggggacagtg ggtatatagg gtgtggaggg 5100
   atggagatat gggggacata agcggatatg atgtgaaggg actcatagga caaggggggc 5160
   aggagcggat ggggggatat tgaggggcac aggtgaggga acatgagaag attgggtatg 5220
   gagggacaca agcgtgggga tgtgggcagt aggatatgga ggggcacatg gggtactggg 5280
   gacgtgagaa ggtatggcat ggagggaccg cacaaatggg gccttgagga gttggggtat 5340
   ggagggacag tgaagggaca cgcgggatat gggggcagga gcagagtatg gggccatgag 5400
   aagatacgga ggaactggtg ggatatgggg gatgtggaca agtggggagt gcagggactc 5460
   cactatgggc aacgtgaaca gaagggatat ggagagaccc cggaaggaca aatgggatat 5520
   gggggacagg agaagacgtg gtacgttctg agcacaggtg tggggattgt gggggggggt 5580
   atggagggac acggccatgg gggcaggtag ggcaatagtg aggggcgcag gtgaggggag 5640
   cagacccgga atggggacgt gagaagatag gagatggagg gacgtatggg atgtggggaa 5700
   cgggagatgg tagggtgagg agggacacgg ggggggggcg acatgaggaa agtgggtgag 5760
   atggaaggac gtggagggtt tgggggggac gacggaggca ggagtagggg gcagtcgcgg 5820
   gacgtggcat atggggggac acaggagcgt tacgtgcaga cagaggagcg cagaggggca 5880
   ccagggctgt ggggcacacg ggtggagctg tgggagcggg tgggggtccc atgggggtgt 5940
   ggaggggtgt gggatgggga gtacgtcacg aggaggggga acatgagaag ctatggggca 6000
   atatgaggat ctggggggca caagaaggag accaggagcg acagagggca gtgataggac 6060
   cccactcccc cccccccgga ccccactttg tggctgtggg gatcccagtg cagcagccga 6120
   tgggctgagc accgcagcac agcccacaga gcctcccgcc ccccccgccc cccctcgcag 6180
   cccccgctta tctctgtgct gcagcccggc tgctgcccgc cgctgagtaa ggggacgctg 6240
   cgagcggctc tgggggggct gcggggactg cgctgcctgc actgcagtgc gcgcccccct 6300
   tccgtggggt gccccacccc cgtcccaaat ttgaatccac cgcaccccaa gagctggcaa 6360
   atcagcacag gggtggggat ggatgtggac aatgaggggg gggggggggt ctgggctggc 6420
   acccctacga gccctctggg tctgggggac cactcagccc cccacccccc atagcatcac 6480
   tggtgtgtta tgggggcccc ctcagccccc catcccgcat agcatcacca atgtgataat 6540
   ggggtacccc acagtgcccc attccccata gcatcaccaa tgtgataatg gagtacccct 6600
   cagcccccca cgccccatag catcaccagt gtggtactgg ggaaccccac agtgccccat 6660
   tccccatagc atcatcaatg tggtaccggg gggaccccac agccccccat ccccccatag 6720
   catcatcaat gtgataatgg ggtaccccac agtgccccat cccccatagc atcatcagtg 6780
   tgatactggg gtaccccaga gccccccatc cccaccatag catcactgat gtggtatgga 6840
   ggccccccca gccccccnnn nnnnnnnccc ccacccccca tagcatcacc aatgtgacac 6900
   aggggtaccc cactgccccc ataacatcac caatgtgaca caggggcgcc ccacagcccc 6960
   catagcatca ccaawgtgac acaggggcgc ccctcagccc cccatgcccc atagcatcac 7020
   caatgtkaca caggggtacc ccactgcccc cataacatca ccaatgtgac acaggggcgc 7080
   cccacagccc ccatagcatc accaatgtga cacaggggcg ccccactgcc cccatagcat 7140
   caccaatgsg tgtccctgct acccggggct gacgctgctg tccccccysa gggctcatcg 7200
   tgtacctggg catgatggtg ggcgcgttcc tgtggggcgg gctggccgac cgcctgggcc 7260
   gaaggcagtg cctcctcatc tccctctccg tcaacagcgt cttcgccttc ttctcctcct 7320
   tcgtccaggg ctacggcacc ttcctgttct gcaggctgct ctcgggcgtg gggtaagggc 7380
   accccgcggg gtsggagtgc tgacgccccc mctttgtgtc ccccccccca ctgacgcccc 7440
   cctctttgca cccccagcat cggcggttcc atccccatcg tcttctccta cttctccaag 7500
   ttcctggcgc aggaaaagcg cggtgagcac ctgagttggc tctgcatgtt ctggatgatc 7560
   ggcggcatct acgcctccgc catggcctgg gccatcatcc cgcactacgg taagggggcg 7620
   gcaacgcggg gcgtgcacgg agctccgggg gtctcagccc cataactgtg tgtgcacgga 7680
   gctctgtggg gtctcagccc cataaccatg tatgcacgga gctccgtggg tctcagcctc 7740
   ataactgcgt gtgcacggag ctctgtgggt ctcagcccca taactgtgtg tgcacggagc 7800
   tctgtggggt ctcagcccca taaccgcgtg tgcactgagt tctgtggggt ctcagcccca 7860
   taaccgcgtg tgcactgagt tctgtggggt ctcagcccca taactgcgtg tgcactgagt 7920
   tctgtggggt ctcagcccca taactgcgtg tgcatggagc tctgtgggtc tcagccccac 7980
   aactgtgtgt gcacggagct ccgtgggtct cagccccata actgtgtgtg cacggagctc 8040
   cgtgggtctc agccccataa ctgcgtgtgc acggagctcc gtgggtctca gccccataac 8100
   cgcgtgtgca ctgagttctg tggggtctca gccccataac catgtgtgca ctgagctctg 8160
   tggggtctca gccccataac tgtgtgtgca cggagctccg tgggtctcag ccccacaact 8220
   gtgtgtgcac ggagctccgt gggtctcagc cccataaccg cgtgtgcacg gagctccgtg 8280
   ggtctcagcc ccataaccac gtgtgcacag agctccgggg gtctcagccc cataactgcg 8340
   tgtgcacgga gctctgtggg tctcagcccc accgccgtct gtgcaccgag ctctgtgggt 8400
   ctcagctcca taaccatgta tacacggagc tccgtgggtc tcagccccat aaccatgtgt 8460
   gcactgagct ctgtggggtc tcagccccat aactgtgtgt gcacggagct ccgtgggtct 8520
   cagccccata accatgtaag cacggagctc catgggtctc agccccataa ctatgtgtgc 8580
   actgagttct gtggggtctc agccccataa ctgtgtgtgc acggagctcc gtgggtctca 8640
   gccccataac tgtgtgtgca cggagctctg tgggtctcag ccccataacc atgtatgcac 8700
   ggagctccat gggtctcagc cccataactg cgtgtgcact gagttctgtg ggtctcagcc 8760
   ccataaccac gtgtgcacgg agctccgtgg gtctcagccc cataaccatg tgtgcaccga 8820
   gctccgtggg tctcagcccc ataaccatgt gtgcatggag ctctgggggt ctcagcccca 8880
   ccgccatgtg tgcactgagc tctgtaggtc tcagccccat aaccgtgtat gcactgagct 8940
   ctgtgggtct cagccccatc gccgtctgtg cactgaattc tgtggctctc agccccataa 9000
   ccatgtgtgc actgagctcc gtgggtctca gccccataac catgtatgca ctgagctctg 9060
   ggggtctcag ccccataacc gtgtgtgcac cgagctctgt ggggtctcag ctggatcacg 9120
   gtgcatgcac cgagctctgc aaggtctcag tccctctgcc atgcatgcat ggagctccgt 9180
   gggtctcasc cccataaccg tgtgtgcact gagttctgtg gggtctcasc cccataaccg 9240
   tgtgtgcacc gagctctggg gtttcasccc caccgccatg tgtgcactga ggtctgtggg 9300
   tctcagcccc ataaccatgt gtgcacagag ctccgtgggg tctcagcccc ataaccacat 9360
   gtgcactgag ctctgtgggt ctcagcccca taaccatgtg tgcaccgagc tctgtgggtc 9420
   tcagccccac cgccatgtgt gcactgaggt ctgtgggtct cagccccata actgtgtatg 9480
   caccgagctc tgtgggtctc agccccatat ccatgtgtgc actgagctct gtgggtctca 9540
   gccccataac cgtgtatgca ctgagctccg tgggtctcag ccccataacc gtgtgtgcac 9600
   tgagctctgt ggggtctcag ccccataacc acatgtgcac tgagctctgt gggtctcagc 9660
   cccataacca tgtgtgcact gagctctgtg ggtctcagcc ccataaccat gtgtgcactg 9720
   agctctgtgg gtctcagccc catatccatg tgtgcacgga gctctgtggg tctcagcccc 9780
   ataaccatgt gtgcactgag ctctgcagtt tctcagctgg atcaccgtgc atgtaccgat 9840
   ctctgtgggg tctcagaccc tgcgccatgt gtgcaccgag ctctgcaagg tctcagctcc 9900
   caccatgcat gcaccgagct ctgcggtgtc tcagtcgggt ctacatgcat gcaccgagct 9960
   ctgtgggatc tcagccccat aaccatgcat gcaccgagct ctgtggggtc tcagccccat 10020
   aaccatgcat gcaccgagct ctgtggggtc tcagccctgt ccccatgcat gcaccgagct 10080
   ctgtgggatc tcagccctgt ccccatgcat gcaccgagct ctgtggggtc tcagccccat 10140
   aaccatgcat gcaccgagct ctgtgggatc tcagccctgt ccccatgcat gcaccgagct 10200
   ctgtgggatc tcagccctgt ccccatgcat gcaccgagct ctgtggggtc tcagccccat 10260
   aaccatgcat gcaccgagct ctgtggggtc tcagccctgt ccccatgcat gcctcaagtt 10320
   ctgtcggtct cagctccacc gccacgtgtg cctcgagctc agtggggtct cagcttccgc 10380
   catccgtgca ttgagctctg cagggtctca gccccataac cgtgcccctt ccccacaggc 10440
   tggagcttcc agatgggctc ggcgtatcag ttccacagct ggagggtctt cgtcctggtg 10500
   tgcgccttcc cctccgtctt cgccatcggg gcgctgacca ctatgcccga gagcccccgc 10560
   ttcttcctgg aggtcagcgg ggttgggggg cacggccacg tcctgccggg ggctgggggc 10620
   tatgggtggg ggtgggaccc ccacrtgggt gctgggaatg gagmggggac ccccggatgg 10680
   ctgctgaggg tgggaaaggg acccccaaat ggatgttgga gttggggtaa ggacccccag 10740
   gtggatactg ggattgggac cccaggtgag tgctggcggc ggcataggga cccccggggg 10800
   gctgctaagg gtggagtagg gatccctata agggcgctgg cgatgggacg gggaccccca 10860
   cgtggacatg gggatggggc agggatcaca ggtgggtgct gagtgtgagg cagggacccc 10920
   gtgaaggtgc tgggggtggg gcggcgcccc cccaaatggg tgctgggggt ggggcggcgt 10980
   tggtcccagg gtgaccccag gctctgccct gcagaacggc aagcacgacg aggcctggat 11040
   ggtgctgaag caggtgcacg acaccaacat gagggccaag ggccaccccg agagggtctt 11100
   ctcggtgagc tccggccccc ctccagcgct gccaccaacc tttgtccagc gccaccactc 11160
   ccatctgggt accatcgtcc caaagccatc agccccatcc ccgtgccatc agccctaccc 11220
   cagtgccacc agtcccattg aataccccca ccccagtgcc accagtccca ttgaataccc 11280
   ccataccagt gccaccagtc ccccctgaat acccccaccc cagtgccacc agtcccattg 11340
   aataccccca ccccagtgcc accagtccca ttgaacaccc ccaccccagt gccaccagtc 11400
   ccattgaata cccccacccc agtgccacca gtcccattga atacccccat accagtgcca 11460
   ccagtcccat tgaaataccc ccaccccagt gccaccagtc ccattgaata cccccacccc 11520
   agtgccacca gtcccattga atacccccac cccagtgcca ccagtcccat tgaatacccc 11580
   cataccagtg ccaccagtcc cattgaatac cccaccccca ctgccaccag tcccattgaa 11640
   tacccccata ccagtgccac cagtcccnnn nnnnnnntac ccccacccca gtgccaccag 11700
   tcccattgaa tacccccata ccagtgccac cagtcccatt gaataccccc accccagtgc 11760
   caccagtccc attgaatacc cccaccccag tgccaccagt cccatcgaat acccccatcc 11820
   cagtgccacc agtcccattg aataccccca taccagtgcc accagtccca ttgaataccc 11880
   ccataccagt gccaccagtc ccattgaata cccccacccc agtgccacca gtcccattga 11940
   atacccccat accagtgcca ccagtcccat cgaatacccc caccccagtg ccaccagtcc 12000
   cattgaatac ccccacccca gtgccaccag tcccattgaa tacccccacc ccagtgccac 12060
   cagtcccatc cgaatacccc cataccagtg ccaccagtcc cattgaatac ccccacccca 12120
   gtgccaccag tcccattgaa tacccccacc ccagtgccac cagtcccatc cgaatacccc 12180
   cataccagtg ccaccagtcc cattgaatac ccccacccca gtgccaccag tcccattgaa 12240
   tacccccatc ccagtgccac cagtcccatt gaataccccc accccagtgc caccagtccc 12300
   attgaatacc cccaccccag tgccaccagt cccattgaat acccccatac cagtgccacc 12360
   agtcccccct gaataccccc accccagtgc caccagtccc attgaatacc cccaccccag 12420
   tgccaccagt cccatccaaa tacccccacc ccagtgccac cagtcccatc caaatacccc 12480
   caccccagtg ccaccagtcc catccaaata cccccatacc agtgccacca gtcccattga 12540
   atacccccat accagtgcca ccagtcccat tgaatacccc caccccagtg ccaccatccc 12600
   caccccagtg ccataagccc cataccggtg ccacccaccc ccacaccagt gcacatcccc 12660
   ccatgcccgc tgctttccct gccaaccccc acctcggggg tcagcatcgc ctgatggagg 12720
   tggggagggg gcgacagcgg gtggggaaac tgaggcacgg gcgcccacag gtgacccaca 12780
   tcaagaccat caagagggag gacgagctga tcgagatcca ggcggacacg ggcgcgtggc 12840
   accgccgctg gttggtgcgc tgcctcagcc tctcgcagca ggtatggggc cagggcctgc 12900
   cccacagccg tgggtccctg ccccacagcc gtgggtccct gccgtgtccy gtggtcccac 12960
   cyctcatccc tccctgcagg tttggagcaa cttccagcag tgcttcgcgc ccgaataccg 13020
   ccgcatcacc ctgatgatga tggccgtgtg gttcaccatg agcttcaggt ggggattggg 13080
   tcagtccctg gggtgggggc aatgggatgg ggctgggggc tttgtgatgg gaacaggaga 13140
   gaggggatgg ggctgggggc attgggatgg gtacaggaga gaggggatgg gggtgggggc 13200
   aatggggtgg ggacaggagt gatgggatgg ggctgggggc attgggatgg gaacaggagt 13260
   gatgggatgg ggctgggggc aatggggtgg gtacaggaaa ggatatggga ctgggggcac 13320
   tgggatggga acaggagtga tgggatgggg ctgggggcaa tggggtgggt acaggagtga 13380
   tgggatgggg gcaatgggat ggggctgggg gcattgggac aggaacagga gcaatggaac 13440
   tggcatggaa atggggctgg gggcaatggg gtgggaacag gagtgatggg atgggggcaa 13500
   tgggatgggg ctgggggcat tgggatggga acaggagtga tgggatgggg ctgggggcaa 13560
   tggggtgggt acaggagaga tgggatgggg gcaatgggat ggggctgggg gcattgggat 13620
   gggaacagaa gcaatggaac tggaatagga atggggctgg gggtaaatgg atgggaacag 13680
   gagcgatggg atggggctgg ggacaacggg acagggctgg gggcattggg atggggctgg 13740
   aaatgaggct gggagcaatg ggatggggct gggggcattg agatggggac aggagcaatg 13800
   gaactggact gggaatgggg gctgggggta atgggactgg gctgatgata ctggtatggg 13860
   accggtggtg ttgggatgag ggtaacagcg ctgggatggg gctgatggca cccggacagg 13920
   gctgacagtg ttgggacaag aatggtggta ccggtatggg agctgggggc tctgggactg 13980
   ggctgatggc actgggatgg aagtggtggt accagtatgg gggctggtgg cgtcaggatg 14040
   gggctgctgc taccactgga agtgggtggc accggtatgg gaccgatggc agcagggctg 14100
   gatgagtggc tctggggtgg gactggtggt gctggggtgg ggataatggc aactggacga 14160
   cggtgatggc attgaggtgg ggcggggggc accgcggtga ggttgacggc actgggattg 14220
   ggcgggtggt aatggtatgg gggctggggg ctctgggatg gcgctgatgg cgttgggacg 14280
   ggggcactgg gatgagggtg atggcactgg gatgggactg gtggtaccgg tatgggggtg 14340
   ggtgacgccg gcatggtgcc gatgggatcg ggatggggct gggagcatcg gaacggggct 14400
   ggtgaccccg ggatagggtg ggtggctctg ggatggagct gacggtgctg ggctggggct 14460
   gatggcggcg gtacggtggt gggtggctga tagtagtggg agggggttgg tggcactggg 14520
   atgggggtgg gcagcagcag aacggggctg gtggcgctgg gacggagccg ggtgcacggt 14580
   gccaggctca gcccctctca ccccgcagtt actacgggct gacggtgtgg ttcccggaca 14640
   tgatcaaaca cctgcagaac atcgactacg cctcacgcac caaactgttc acccgcgaga 14700
   aggtccggca cttcaccttc aacttcaccc tggagaacca gatccaccag ggcggggagt 14760
   acttcaacga caagtgggtg gtgggggcag tggggtgcgg cggggggggc tgggatggga 14820
   atggtggtgc tgggatgggg ctgacggtac cagtatgggc tcggtggtgg cactgggatg 14880
   gggatggggg cactgggatg gagctgaggg ctttgggaca gggctggggg agtttaggat 14940
   gggctctgaa atggggctgg ggacactggg atggaaactg aagcaatgaa atggggctgg 15000
   gggcattggg acatggttgg gggctctggg atggggctgg gggcattagg atggggttgg 15060
   gaggctctgn nnnnnnnnnc tgggatgggg ctgggggcat tgggacgggt tgagggctct 15120
   gggatggggc tgggggcatt gggatgaggc tagaggcatt aggatagggc tgggggtgtt 15180
   gagatggggc tggaggcact ggggtggata cagaagcaat gagacgggtg tgggggcact 15240
   gggatggggg tgttgggatg gagttgatgg taccagtaca ggctgggtgg cactgggatg 15300
   ggactgggag cactgggatg gacacagaag caatgggacg atacaggggg tgttgggatg 15360
   aggctgatgg tagcagtatg gggtgagtgg cactgggaca aggctggtgg cagtgggatg 15420
   gggctggtgg tgcccacatg gagctgatgg cgccggctgt cccccccccc cccccccccc 15480
   aggttcattg ggctgaagat gaagtcggtg acgttcgagg actctctgtt cgaggactgc 15540
   tacttcgagg acatcacctc cagcaacacc ttcttcaaga actgcacctt catctccacc 15600
   atgttctaca acacaggttt gggagggggg gagcgaggtg ggacaggggg aaacgggggc 15660
   agggagggca gcccccgacc ccccgccccc ccccttccct ctgcctcccc ccagacctct 15720
   tcgagtacaa gttcatcaac agccgcgtgc tgaacagcac cttcatgcac aacaaggagg 15780
   gctgccagct ggacttcagc gatgacaaca acgcctacat gatctacttt gtcagcttcc 15840
   tcggcaccct ggccgtgctg cccgggaaca tcgtctcagc gctgctcatg gacaagatcg 15900
   gccgcctccg catgctgggt gggtgcagct gcccccctcc cctgcatcca tttccccctt 15960
   ccctctccca ttttcccctc ttctgactca gcgtgccccc tgctgcctca gtttccccct 16020
   ttcctcccct gttttcccat ctcctgcctc agtttcccca tatgtacctc aatttcccta 16080
   tatgtgcctc agtttcccta tatgtgcctc agtttccctc tctgctgctt cattttcccc 16140
   cctctgctgc ctcagtttcc ccttcctccc gtttccctct cctgcctcac tgttccactc 16200
   tactgcctca gtttcccctc tcctccccca ttttcccctc tcctgcctca gtttcccctc 16260
   tgcagctcca tttcccaccc cacagcccca tttcccccct ctcctgtccc agcgcccccc 16320
   tttcggccgc ctccattccc ccctctcctc ccctaccttt cccccttctg cctcagtttc 16380
   ccccctgacg ctctgtcccc tggcagccgg ctccagcgtg atgtcctgtg tgagctgctt 16440
   cttcctgtcc ttcgggaaca gcgaatctgc catgattgca ctgctctgcc tcttcggtgg 16500
   cgtcagcatc gcctcctgga ac 16522